# EUROPÄISCHE PATENTSCHRIFT

(11) **EP 2 134 807 B1**
(45) Veröffentlichungstag und Bekanntmachung des Hinweises auf die Patenterteilung: **31.08.2011**
(21) Anmeldenummer: 08735828.9
(22) Anmeldetag: 04.04.2008
(51) Int. Cl.: C09K 11/06

(54) **IRIDIUM-TRIPHENYLEN-KOMPLEXE UND DEREN VERWENDUNG IN OLEDS**
IRIDIUM-TRIPHENYLENE COMPLEXES AND THEIR USE IN OLEDS
TRIPHÉNYLÈNE COMPLEXES D'IRIDIUM ET LEUR UTILISATION DANS DES OLEDS

(30) Priorität: 04.04.2007 EP 07105649
(43) Veröffentlichungstag der Anmeldung: 23.12.2009
(73) Patentinhaber: BASF SE, 67056 Ludwigshafen (DE)
(72) Erfinder: BOERNER, Herbert Friedrich, 52076 Aachen (DE); LOEBL, Hans-Peter, 52156 Monschgau-Imgenbroich (DE); SALBECK, Josef, 43260 Kaufungen (DE); POPOVA, Elena, 34132 Kassel (DE)
(86) Internationale Anmeldenummer: PCT/EP2008/054087
(87) Internationale Veröffentlichungsnummer: WO 2008/122603

(56) Entgegenhaltungen:
- WO-A-2006/093466
- US-A1- 2006 036 097
- MARAPPAN VELUSAMY ET AL: "Synthesis, structure and electroluminescent properies of cyclometalated iridium complexes possessing sterically hindered ligands" DALTON TRANSACTIONS,, 30. März 2007 (2007-03-30), Seiten 3025-3034, XP002490863

## Beschreibung

Die vorliegende Erfindung betrifft metallorganische Komplexe, die mindestens einen Liganden tragen, der eine Einheit mit einer Triplett-Energie von mindestens 22.000 cm⁻¹ aufweist, ein Verfahren zur Herstellung der metallorganischen Komplexe, eine Mischung enthaltend mindestens einen erfindungsgemäßen metallorganischen Komplex, die Verwendung der metallorganischen Komplexe oder der Mischung in organischen Leuchtdioden, wobei die metallorganischen Komplexe bevorzugt als Emittermaterialien eingesetzt werden, sowie spezielle Stickstoff- oder Phosphorsubstituierte Triphenylenderivate und ein Verfahren zu deren Herstellung.

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen und als besonders effiziente Lichtquelle. Aufgrund der sehr kompakten Bauweise und des intrinsisch niedrigen Stromverbrauchs eignen sich Vorrichtungen enthaltend OLEDs insbesondere für mobile Anwendungen, zum Beispiel für Anwendungen in Handys, Laptops, Digitialkameras, usw.

Die Grundprinzipien der Funktionsweise von OLEDs sowie geeignete Aufbauten (Schichten) von OLEDs sind dem Fachmann bekannt und zum Beispiel in WO 2005/113704 und der darin zitierten Literatur genannt. Als Licht-emittierende Materialien (Emitter) können neben fluoreszierenden Materialien (Fluoreszenz-Emitter) phosphoreszierende Materialien (Phosphoreszenz-Emitter) eingesetzt werden. Bei den Phosphoreszenz-Emittern handelt es sich üblicherweise um metallorganische Komplexe, die im Gegensatz zu den Fluoreszenz-Emittern, die eine Singulett-Emission zeigen, eine Triplett-Emission zeigen (Triplett-Emitter) (M. A. Baldow et al., Appl. Phys. Lett. 1999, 75, 4 bis 6).

Aus quantenmechanischen Gründen ist bei der Verwendung der Triplett-Emitter (Phosphoreszenz-Emitter) eine bis zu vierfache Quanten-, Energie- und Leistungeffizienz möglich. Um die Vorteile des Einsatzes der metallorganischen Triplett-Emitter in die Praxis umzusetzen, ist es wünschenswert, Emittermaterialien bereitzustellen, die sich durch eine gute Stabilität, eine hohe Lumineszenz-Effizienz, eine hohe Farbenreinheit und geeignete Löslichkeiten auszeichnen.

Im Stand der Technik werden zahlreiche verschiedene Materialien für den Einsatz als Emittermaterialien in OLEDs vorgeschlagen. Unter der vorgeschlagenen Materialien sind auch Übergangsmetallkomplexe, die Phosphoreszenz zeigen.

So betrifft US 2002/0034656 A1 eine Licht-emittierende Schicht eines OLEDs, die eine phosphoreszente organometallische Verbindung aufweist, zur Erhöhung der Quanteneffizienz des OLEDs. Als Emittermaterialien sind gemäß US 2002/0034656 A1 phosphoreszierende organometallische Komplexe von Platium, Iridium oder Osmium besonders geeignet, wobei ganz besonders bevorzugt cyclometallierte phosphoreszierende Platin-, Iridium- oder Osmium-Komplexe eingesetzt werden. Als Beispiele für geeignete phosphoreszierende Übergangsmetallkomplexe werden Ir(ppy)₃ sowie Platin(II)-Komplexe mit Bis[2-(2-Phenyl)pyridinato-N,C2], Bis[2(2'-Thienyl)pyridinato-N,C3] oder Bis[Benzo-(h)chinolinato-N,C] genannt.

Des Weiteren sind als Emittermaterialien geeignete phosphoreszierende Ir-Komplexe der allgemeinen Formeln 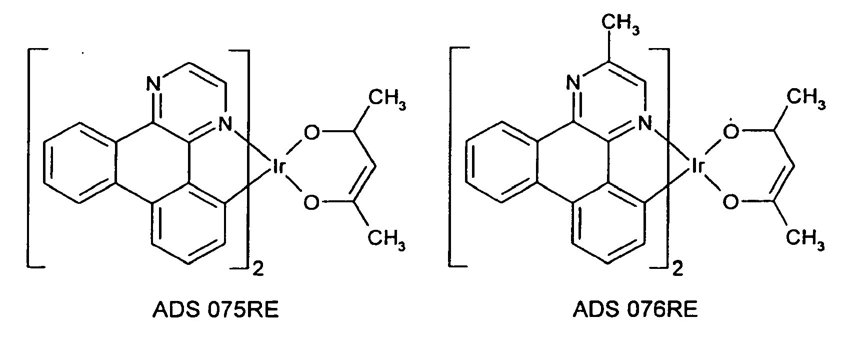 von American Dyesource (www.adsdyes.com) bekannt (Verbindungen ADS 075RE und ADS 076RE). Diese Komplexe zeigen jedoch eine starke Rotverschiebung der Emission.

WO 2006/093466 betrifft aus Lösung prozessierbare phosphorezente Licht emittierende Materialien und organische Leuchtdioden, die diese enthalten. Die Licht emittierenden Materialien sind phosphoreszente metallorganische Komplexe. Unter anderem sind in den Beispielen 8, 9 und 10 in diesem Dokument die Liganden D, E und F als geeignete Liganden der metallorganischen Komplexe genannt:

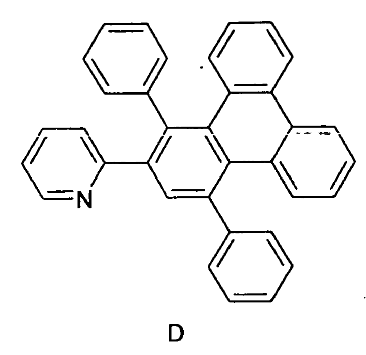

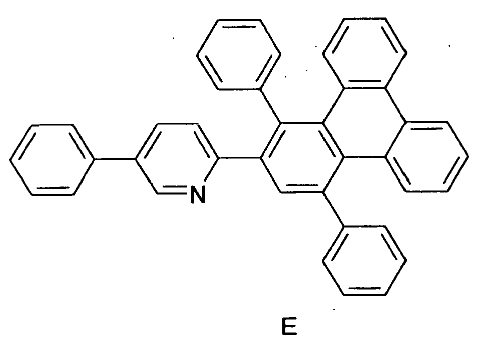

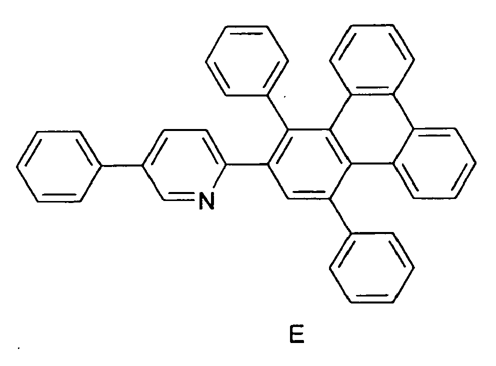

Diese Liganden werden gemäß den Beispielen 20, 21 und 22 in den Ir-Komplexen D₂Ir(acac), E₂Ir(acac) und F₂Ir(acac) eingesetzt.

M. Velusamy et al., Dalton Trans. 2007, 3025 bis 3034 betrifft cyclometallierte Ir-Kmplexe, und deren Eignung für den Einsatz als Licht emittierende Materialien in organischen Leuchtdioden.

Aufgabe der vorliegenden Erfindung ist die Bereitstellung von Emittermaterialien für OLEDs, die eine gute thermische Stabilität zeigen und zur Herstellung von OLEDs mit guter Effizienz und hoher Farbenreinheit geeignet sind.

Diese Aufgabe wird gelöst durch die Bereitstellung von metallorganischen Komplexen der allgemeinen Formel (I)

M[L₁]_{q}[L₂]ᵣ[L₃]ₛ (I)

worin bedeuten
- M: Ir;
- L₁: monoanionischer bidentater Ligand, basierend auf einem Triphenylderivat der Formel (IIa) 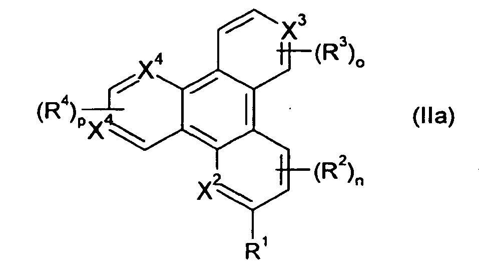 worin bedeuten:
R¹ Rest der Formel 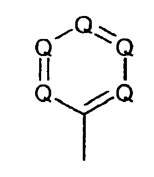 wobei Q jeweils unabhängig voneinander CR^{a} oder N bedeutet, wobei mindestens eine Gruppe Q in ortho-Stellung zu der Bindungsstelle N bedeutet. Im Allgemeinen enthält der vorstehend genannte Rest R¹ insgesamt 1, 2, 3 oder 4 N-Atome, bevorzugt 1, 2 oder 3 N-Atome, besonders bevorzugt 1 oder 2 N-Atome. Die weiteren Ringglieder in dem vorstehend genannten Rest R¹ sind C-Atome. R^{a} bedeutet unabhängig voneinander Wasserstoff, Alkyl, Cycloalkyl, Heterocycloalkyl, Aryl, Heteroaryl, Amino oder eine Gruppe mit Donor- oder Akzeptorwirkung;
oder ein Rest der Formeln 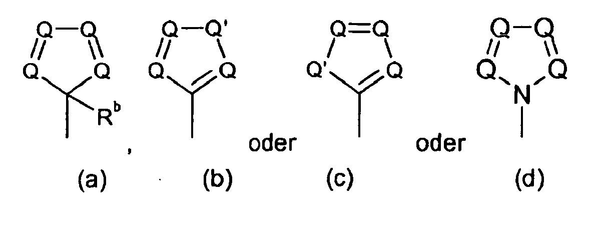 wobei Q jeweils unabhängig voneinander CR^{a} oder N bedeutet, wobei mindestens eine Gruppe Q in ortho-Stellung zu der Bindungsstelle N bedeutet, und Q' CR^{a}₂, O, S oder NR^{c} bedeutet. Im Allgemeinen enthält der vorstehend genannte Reste R¹ (a) insgesamt 1, 2, 3 oder 4 N-Atome, bevorzugt 1, 2 oder 3 N-Atome, besonders bevorzugt 1 oder 2 N-Atome. Der vorstehend genannte Rest R¹ (b) enthält im Allgemeinen entweder insgesamt 1, 2, 3 oder 4 N-Atome, bevorzugt 1, 2 oder 3 N-Atome, besonders bevorzugt 1 oder 2 N-Atome. Es ist ebenfalls möglich, dass der Rest R¹ (b) insgesamt 1, 2 oder 3 N-Atome und 1 O- oder 1 S-Atom, bevorzugt 1 oder 2 N-Atome und 1 O- oder 1 S-Atom, besonders bevorzugt 1 N-Atom und 1 O- oder 1 S-Atom enthält. Der vorstehend genannte Rest R¹ (d) enthält im Allgemeinen insgesamt 1, 2, 3 oder 4 N-Atome, bevorzugt 1, 2 oder 3 N-Atome, besonders bevorzugt 1 oder 2 N-Atome. Die weiteren Ringglieder in den vorstehend genannten Resten R¹ sind C-Atome. R^{a}, R^{b} und R^{c} bedeuten unabhängig voneinander Wasserstoff, Alkyl, Cycloalkyl, Heterocycloalkyl, Aryl, Heteroaryl, Amino, CF₃, CN, Alkoxy oder F.
- R², R³, R⁴: unabhängig voneinander C₁-C₂₀-Alkyl, C₀-_{C20}-Alkylen-C₃-C₈--cycloalkyl, C₀-C₂₀- Alkylen-heterocycloalkyl mit 3 bis 18 Ringatomen, C₀-C₂₀-Alkylen-C₁-C₂₀-alkoxy, C₀-C₂₀-Alkylen-C₆-C₁₈-aryloxy, C₀-C₂₀-Alkylen-C₆-C₁₈-aryl, C₀-C₂₀-Alkylen- heteroaryl mit 5 bis 18 Ringatomen, wobei die vorstehend genannten Reste mit Hydroxy, Halogen, Pseudohalogen, Alkyl, Cycloalkyl, Heterocycloalkyl, Aryl, He- teroaryl, Amino, -C(O)R', -C(O)OR", -OC(O)R"', -OC(O)OR"", wobei R', R", R'" und R"" unabhängig voneinander Wasserstoff, Alkyl, Cycloalkyl, Heterocycloalkyl, Aryl, Heteroaryl oder Amino bedeuten, substituiert oder unsubstituiert sein können; Hydroxy, Halogen, Pseudohalogen, Phosphonat, Phosphat, Phosphin, Phosphinoxid, Phosphoryl, Sulfonyl, Sulfonat, Sulfat, Amino, Polyether, Silyl-C₁-C₂₀-alkyl, Silyl-C₀-C₂₀-Alkylen-C₆-C₁₈-aryl, Silyl-C₀-C₂₀- Alkylen-C₁C₂₀-alkoxy, -C(O)R', -C(O)OR", -OC(O)R"', -OC(O)OR"", wobei R', R", R"' und R"" unabhängig voneinander Wasserstoff, Alkyl, Cycloalkyl, Heterocycloalkyl, Aryl, Heteroaryl oder Amino bedeuten; bevorzugt bedeuten R², R³ und R⁴ unabhängig voneinander C₁-C₂₀-Alkyl, bevor- zugt C₁-C₈-Alkyl; C₀-C₂₀-Alkylen-C₃-C₁₈-Cycloalkyl, bevorzugt C₀-C₆-Alkylen-C₅- C₆-Cycloalkyl; C₀-C₂₀-Alkylen-hetereocycloalkyl mit 3 - 18 Ringatomen, bevor- zugt C₀-C₆-Alkylenheterocycloalkyl mit 5 oder 6 Ringatomen; C₀-C₂₀-Alkylen-C₁- C₂₀-alkoxy, bevorzugt C₀-C₆-Alkylen-C₁-C₈-alkoxy; C₀-C₂₀-Alkylen-C₆-C₁₈-aryloxy, bevorzugt C₀-C₈-Alkylen-C₆-aryloxy; C₀-C₂₀-Alkylen-C₆-C₁₈-aryl, bevorzugt C₀-C₆- Alkylen-C₆-aryl; C₀-C₂₀-Alkylen-heteroaryl mit 5 - 18 Ringatomen, bevorzugt C₀- C₆-Alkylen-heteroaryl mit 5 oder 6 Ringatomen, wobei die genannten Reste unsubstituiert oder substituiert sein können, wobei bevorzugte Substituenten Al- kyl, bevorzugt C₁-C₈-Alkyl; Alkoxy, bevorzugt C₁-C₈-Alkoxy; Halogen, bevorzugt F, Cl, Br, I, besonders bevorzugt F, Cl; oder Pseudohalogen, bevorzugt CN, SCN, OCN, N₃, CNO, SeCN, besonders bevorzugt CN, SCN;
- o: 0 bis 3, wobei die Reste R³ bei o > 1 gleich oder verschieden sein können;
- p: 0 bis 2, wobei die Reste R⁴ bei p > 1 gleich oder verschieden sein können;
- n: 0;
- X²: CH;
- X³: N, CH oder CR³;
- X⁴: unabhängig voneinander N, CH oder CR⁴;
L₂ mono- oder dianionischer Ligand, der mono- oder bidentat sein kann;
L₃ neutraler mono- oder bidentater Ligand;
q Zahl der Liganden L₁, wobei q 1, 2 oder 3 ist und die Liganden L₁ bei q > 1 gleich oder verschieden sein können;
r Zahl der Liganden L₂, wobei r 0 bis 4 ist und die Liganden L₂ bei r > 1 gleich oder verschieden sein können;
s Zahl der Liganden L₃, wobei s 0 bis 4 ist und die Liganden L3 bei s > 1 gleich oder verschieden sein können;
wobei die Summe q + r + s von der Oxidationsstufe und Koordinationszahl des eingesetzten Metalls M und von der Zähnigkeit der Liganden L₁, L₂ und L₃ sowie von der Ladung der Liganden L₁ und L₂ abhängig ist.

Die metallorganischen Komplexe der Formel (I) gemäß der vorliegenden Erfindung zeichnen sich bei Einsatz in OLEDs durch hervorragende Effizienzen aus, insbesondere deswegen, da sie in der Licht-emittierenden Schicht eines OLEDs in hoher Konzentration vorliegen können, wobei die Bildung von Dimeren und somit das Quenchen des angeregten Zustandes unterdrückt werden kann. Des Weiteren können bei Einsatz der erfindungsgemäßen metallorganischen Komplexe Emissionen mit hoher Farbenreinheit erzielt werden, und die metallorganischen Komplexe gemäß der vorliegenden Erfindung weisen eine hohe thermische Stabilität auf.

Die erfindungsgemäßen metallorganischen Komplexe enthalten mindestens eine auf Triphenylen oder einem Derivat davon basierende Einheit mit einer Triplett-Energie von mindestens 22.000 cm⁻¹ (ermittelt durch Tieftemperatur-Photolumineszenz-Messungen), bevorzugt mit einer Triplett-Energie von 22.000 cm⁻¹ bis 28230 cm⁻¹, besonders bevorzugt 22000 bis 25000 cm⁻¹. Unter der Triplett-Energie wird im Sinne der vorliegenden Anmeldung die Energie des ersten Triplettniveaus verstanden.

Der Ligand L₁ in den erfindungsgemäßen metallorganischen Komplexe der Formel (I) weist bevorzugt eine Triplett-Energie von mindestens 16.000 cm⁻¹, bevorzugt 16.000 cm⁻¹ bis 19.500 cm⁻¹ besonders bevorzugt 16000 bis 18500 cm⁻¹ auf.

Die erfindungsgemäßen metallorganischen Komplexe zeigen im Allgemeinen Elektrolumineszenz im sichtbaren Bereich des elektromagnetischen Spektrums, bevorzugt von 400 nm bis 800 nm, besonders bevorzugt von 450 nm bis 800 nm, ganz besonders bevorzugt von 490 nm bis 750 nm.

Der Rest R¹ in dem Liganden L₁ basierend auf einer Verbindung der Formel (II) ist erfindungsgemäß ein N enthaltender Rest. Insbesondere ganz besonders bevorzugt ist der Rest R¹ ein Pyridyl- oder Benzothiazylrest oder ein Triazolyl-, ein Isoxazolyl- oder ein Pyrazolylrest, der substituiert oder unsubstituiert sein kann. Geeignete Substituenten des Restes R¹ sind die nachstehend genannten geeigneten Substituenten. In einer ganz besonders bevorzugten Ausführungsform ist der Rest R¹ unsubstituiert, das heißt alle substituierbaren Positionen des Rests R¹ sind mit Wasserstoffatomen substituiert.

Im Sinne der vorliegenden Anmeldung haben die Begriffe Alkyl, Alkylen, Cycloalkyl, Heterocycloalkyl, Alkoxy; Aryloxy, Aryl, Heteroaryl, Halogen, Pseudohalogen, Amino, Phosphonat, Phosphat, Phosphin, Phosphinoxid, Phosphoryl, Sulphonyl, Sulfonat, Sulfat, Polyether, Silylalkyl, Silylalkylenaryl und Silylalkylenalkoxy im Allgemeinen die folgenden Bedeutungen, wobei besonders bevorzugte Bedeutungen in den spezifischen Definitionen der einzelnen Reste genannt sind:

Unter Alkyl ist ein Rest mit 1 bis 20 Kohlenstoffatomen, bevorzugt 1 bis 10 Kohlenstoffatomen, besonders bevorzugt 1 bis 8 Kohlenstoffatomen in der längsten Alkylkette zu verstehen. Dieser Alkylrest kann verzweigt oder unverzweigt sein und gegebenenfalls mit einem oder mehreren Heteroatomen, z: B. Si, N, oder S, bevorzugt N, O oder S unterbrochen sein. Des Weiteren kann der Alkylrest mit einem oder mehreren bezüglich des nachstehend genannten Arylsubstituenten genannten Substituenten substituiert sein. Besonders bevorzugt sind die Alkylreste ausgewählt aus der Gruppe bestehend aus Methyl, Ethyl, iso-Propyl, n-Propyl, n-Butyl, sec-Butyl, iso-Butyl, tert-Butyl, sowie CF₃.

Unter einem Cycloalkylrest ist ein cyclischer Alkylrest mit einem Grundgerüst von drei bis 18 Kohlenstoffatomen, bevorzugt 5 bis 8 Kohlenstoffatomen, besonders bevorzugt 5 oder 6 Kohlenstoffatomen zu verstehen. Geeignete Grundgerüste sind z. B. Cyclopentyl oder Cyclohexyl. Das Grundgerüst des Cycloalkylrests kann unsubstituiert sein (d. h., dass alle Kohlenstoffatome, die substituierbar sind, Wasserstoffatome tragen), oder an einer, mehreren oder allen substituierbaren Positionen des Grundgerüsts substituiert sein. Geeignete Substituenten sind die nachstehend bezüglich der Arylreste genannten Substituenten. Besonders bevorzugte Cycloalkylreste sind Cyclohexyl und Cyclopentyl.

Unter einem Heterocycloalkylrest ist ein Rest mit 3 bis 18 Ringatomen im Grundgerüst, bevorzugt 5 oder 6 Ringatomen, zu verstehen. Daneben enthält der Heterocycloalkylrest mindestens ein Heteroatom ausgewählt aus der Gruppe bestehend aus N, O und S. Der Heterocycloalkylrest kann an einer, mehreren oder allen substituierbaren Positionen des Grundgerüsts substituiert sein. Geeignete Substituenten sind die bezüglich der Arylreste genannten Substituenten.

Unter Aryl ist ein Rest mit einem Grundgerüst von 6 bis 18, bevorzugt 6 bis 10, besonders bevorzugt 6 Kohlenstoffatomen zu verstehen, der aus einem aromatischen Ring oder mehreren kondensierten aromatischen Ringen aufgebaut ist. Geeignete Grundgerüste sind z. B. Phenyl, Naphtyl, Anthracenyl oder Phenanthrenyl. Dieses Grundgerüst kann unsubstituiert sein (d. h., dass alle Kohlenstoffatome, die substituierbar sind, Wasserstoffatome tragen), oder an einer, mehreren oder allen substituierbaren Positionen des Grundgerüsts substituiert sein. Geeignete Substituenten sind z. B. die vorstehend genannten Alkylreste, Arylreste, bevorzugt C₆-Arylreste, die wiederum substituiert oder unsubstituiert sein können, Heteroarylreste, bevorzugt Heteroarylreste, die mindestens ein Stickstoffatom enthalten, besonders bevorzugt Pyridylreste oder Gruppen mit Donor- oder Akzeptorwirkung. Geeignete Gruppen mit Donor- oder Akzeptorwirkung sind nachstehend genannt. Ganz besonders bevorzugt tragen die Arylreste Subsituenten ausgewählt aus der Gruppe bestehend aus Methyl, F, Cl, CN, Aryloxy und Alkoxy. Bevorzugt ist der Arylrest ein C₆-C₁₈-Arylrest, besonders bevorzugt ein C₆-C₁₀-Arylreste, ganz besonders bevorzugt ein C₆-Arylrest, der mit keinem, einem oder zwei der vorstehend genannten Substituenten substituiert ist, wobei im Falle des C₆-Arylrests, der eine Substituent in ortho-, meta- oder para-Position zur weiteren Verknüpfungsstelle des Arylrests angeordnet ist, und - im Falle von zwei Substituenten - diese jeweils in meta-Position oder ortho-Position zur weiteren Verknüpfungsstelle des Arylrests angeordnet sein können, oder ein Rest in ortho-Position und ein Rest in meta-Position angeordnet ist oder ein Rest in ortho- oder meta-Position und der weitere Rest in para-Position angeordnet ist.

Unter einem Heteroarylrest ist ein Rest zu verstehen, der 5 bis 18 Ringatome, bevorzugt 5 oder 6 Ringatome, aufweist. Mindestens eines der Ringatome ist ein Heteroatom, wobei bevorzugte Heteroatome ausgewählt sind aus der Gruppe bestehend aus N, O und S. Bevorzugt weist der Heteroarylrest ein oder zwei Heteroatome auf. Besonders bevorzugt ist das Grundgerüst ausgewählt aus Carbazol, Pyridin, Pyrrol, Furan, Pyrazol, Imidazol und Thiophen. Das Grundgerüst kann an einer, mehreren oder allen substituierbaren Positionen des Grundgerüsts substituiert sein. Geeignete Substituenten sind dieselben, die bereits bezüglich der Arylgruppe genannt wurden.

Unter einer Alkoxygruppe ist eine O-Alkylgruppe zu verstehen, wobei der Alkylrest die vorstehend genannten Bedeutungen aufweisen kann. Ein Beispiel für eine bevorzugte Alkoxygruppe ist OMe.

Unter einer Aryloxygruppe ist eine O-Arylgruppe zu verstehen, wobei geeignete Arylgruppen vorstehend genannt sind. Ein Beispiel für eine geeignete Aryloxygruppe ist eine Phenoxygruppe.

Unter dem Ausdruck C₀-C₂₀-Alkylen ist zu verstehen, dass die entsprechenden Reste oder Gruppen direkt mit dem Grundgerüst verbunden sein können (C₀-Alkylen) oder über eine Alkylen-Gruppe mit 1 bis 20 Kohlenstoffatomen, bevorzugt 1 bis 10, besonders bevorzugt 1 bis 6, ganz besonders bevorzugt 1 oder 2 Kohlenstoffatomen, mit dem Grundgerüst verbunden sein können (C₁-C₂₀-Alkylen, bevorzugt C₁-C₁₀-Alkylen, besonders bevorzugt C₁-C₆-Alkylen, ganz besonders bevorzugt C₁-C₂-Alkylen). Der Alkylenrest entspricht den vorstehend genannten Alkylresten mit dem Unterschied, dass der Alkylenrest zwei Bindungsstellen zu weiteren Gruppen aufweist. Beispielsweise sind bevorzugte C₀-C₂₀-Alkylen-C₆-C₁₈-arylreste Benzylreste.

Unter einer Gruppe mit Donor- oder Akzeptorwirkung sind im Sinne der vorliegenden Anmeldung die folgenden Gruppen zu verstehen:
Unter Gruppen mit Donorwirkung sind Gruppen zu verstehen, die einen +|- und/oder +M-Effekt aufweisen, und unter Gruppen mit Akzeptorwirkung sind Gruppen zu verstehen, die einen -|- und/oder -M-Effekt aufweisen. Geeignete Gruppen mit Donor- oder Akzeptorwirkung sind Halogenreste, bevorzugt F, Cl, Br, I besonders bevorzugt F, Cl, Alkoxyreste, Aryloxyreste, Carbonylreste, Esterreste, sowohl Oxycarbonyl als auch Carbonyloxy, Aminreste, Amidreste, CH₂F-Gruppen, CHF₂-Gruppen, CF₃-Gruppen, CN-Gruppen, Thio-Gruppen, Sulfonsäure-Gruppen, Sulfonsäureester-Gruppen, Boronsäure-Gruppen, Boronsäureester-Gruppen, Phosphonsäure-Gruppen, Phosphonsäureester-Gruppen, Phosphinreste, Sulfoxidreste, Sulfonylreste, Sulfidreste, NitroGruppen, OCN, Boranreste, Silyl-Gruppen, Stannatreste, Imino-Gruppen, Hydrazinreste, Hydrazolreste, Oximreste, Nitroso-Gruppen, Diazo-Gruppen, Phosphinoxid-Gruppen, Hydroxy-Gruppen oder SCN-Gruppen. Ganz besonders bevorzugt sind F, Cl, CN, Aryloxy und Alkoxy.

Unter Pseudohalogen ist eine Gruppe ausgewählt aus CN, SCN, OCN, N₃, CNO und SeCN, bevorzugt CN oder SCN zu verstehen.

Unter Halogen ist eine Gruppe ausgewählt aus F, Cl, Br und I, bevorzugt F oder Cl zu verstehen.

Der Ausdruck "Amino" ist eine Gruppe -NR₂, worin jeder Rest R unabhängig voneinander ausgewählt ist aus Wasserstoff, C₁-C₆-Alkyl, C₁-C₆-Alkylen-C₆H₅ und C₆-C₁₈-Aryl, wobei die beiden Reste R des Weiteren gemeinsam mit dem Stickstoffatom einen 4-bis 6-gliedrigen, bevorzugt 5- bis 6-gliedrigen heterozyklischen Ring bilden können, der gegebenenfalls mit C₁-C₆-Alkylresten substituiert sein kann, bevorzugt C₁-C₆-Alkyl, Benzyl oder Phenyl.

Unter Phosphonat sind Gruppen -P(O)(OR)₂ zu verstehen, worin die Reste R unabhängig voneinander ausgewählt sind aus Wasserstoff, Alkyl und Aryl, bevorzugt Wasserstoff, C₁-C₆-Alkyl, Phenyl und Benzyl. Des Weiteren können die Reste R ein Kation bedeuten, z. B. Na⁺, K⁺, Mg²⁺ und Ca²⁺.

Unter Phosphat ist -OP(O)(OR)₂ zu verstehen, worin die Reste R unabhängig voneinander Wasserstoff, Alkyl oder Aryl bedeuten, bevorzugt Wasserstoff, C₁-C₆-Alkyl, Phenyl oder Benzyl. Des Weiteren können die Reste R ein Kation ausgewählt aus Na⁺, K⁺, Mg²⁺ und Ca²⁺ bedeuten.

Unter Phosphin ist -P(R)₂ zu verstehen, worin die Reste R unabhängig voneinander Wasserstoff, Alkyl oder Aryl, bevorzugt Wasserstoff, C₁-C₆-Alkyl, Phenyl oder Benzyl bedeuten.

Unter Phosphinoxid ist -P(O)R₂ zu verstehen, worin die Reste R unabhängig voneinander Wasserstoff, Alkyl, Aryl oder Amino bedeuten, bevorzugt Wasserstoff, C₁-C₆-Alkyl, Phenyl, Benzyl oder -NR'₂, worin R' jeweils unabhängig voneinander Wasserstoff, Alkyl oder Aryl, bevorzugt Wasserstoff, C₁-C₆-Alkyl, Phenyl oder Benzyl bedeutet.

Unter Sulfonyl ist -S(O)₂R zu verstehen, worin R Wasserstoff, Alkyl, Aryl oder Amino bedeutet, bevorzugt Wasserstoff, C₁-C₆-Alkyl, Phenyl, Benzyl oder -NR'₂, worin R' jeweils unabhängig voneinander Wasserstoff, Alkyl oder Aryl, bevorzugt Wasserstoff, C₁-C₆-Alkyl oder Benzyl bedeutet.

Unter Sulfonat ist -S(O)₂OR zu verstehen, worin R Wasserstoff, Alkyl oder Aryl, bevorzugt Wasserstoff, C₁-C₆-Alkyl, Phenyl oder Benzyl bedeutet. Des Weiteren kann R ein Kation ausgewählt aus Na⁺, K⁺, Mg²⁺ oder Ca²⁺ bedeuten.

Unter Sulfat ist -OS(O)₂OR zu verstehen, worin R Wasserstoff, Alkyl oder Aryl, bevorzugt Wasserstoff, C₁-C₆-Alkyl, Phenyl oder Benzyl bedeutet. Des Weiteren kann R ein Kation ausgewählt aus Na⁺, K⁺, Mg²⁺ oder Ca²⁺ bedeuten.

Unter einem Polyetherrest ist eine Gruppe zu verstehen, ausgewählt aus den Gruppen -(O-CHR)ₙ-OH und -(O-CH₂-CHR)ₙ-H, wobei R unabhängig ausgewählt ist aus Wasserstoff, Alkyl, Aryl, Halogen und n 1 bis 250 bedeutet.

Unter Silyl-C₁-C₂₀-Alkyl ist eine Gruppe SiR₃ zu verstehen, wobei die Reste R Wasserstoff oder Alkyl, bevorzugt C₁-C₆-Alkyl oder Wasserstoff bedeuten.

Unter Silyl-C₀-C₂₀-alkylen-C₆-C₁₈-aryl sind Gruppen -SiR₃ zu verstehen, wobei R unabhängig voneinander ausgewählt ist aus Aryl, bevorzugt C₆-C₁₈-Aryl, besonders bevorzugt Phenyl, wobei die Arylgruppe direkt an das Si gebunden ist (C₀-Alkylen) und Gruppen C₁-C₂₀-Alkylen-aryl, bevorzugt C₁-C₂₀-Alkylen-C₆-C₁₈-aryl, besonders bevorzugt C₁-C₆-Alkylen-phenyl.

Unter einer Gruppe Silyl-C₀-C₂₀-Alkylen-C₁-C₂₀-Alkoxy ist eine Gruppe -Si(OR)₃ zu verstehen, wenn eine C₀-Alkylengruppe vorliegt, wobei der Rest R ein C₁-C₂₀-Alkylrest, bevorzugt ein C₁-C₆-Alkylrest ist. Des Weiteren ist unter der genannten Gruppe eine - Si-C₁-C₂₀-Alkylen-C₁-C₂₀-alkoxy-Gruppe, bevorzugt eine -Si-C₁-C₆-Alkylen-C₁-C₆-alkoxy-Gruppe zu verstehen.

In den Gruppen -C(O)R', -C(O)R", -OC(O)R"', -C(O)OR"" haben R', R", R"' und R"" unabhängig voneinander die Bedeutung Wasserstoff, Alkyl, Cycloalkyl, Heterocycloalkyl, Aryl, Heteroaryl oder Amino, bevorzugt Wasserstoff, C₁-C₆-Alkyl, C₃-C₈-Cycloalkyl, Heterocycloalkyl mit drei bis acht Ringatomen, C₆-C₁₈-Aryl, bevorzugt Phenyl, Heteroaryl mit 5 bis 18 Ringatomen oder Amino wie vorstehend definiert.

Unter einem bidentaten Liganden ist ein Ligand zu verstehen, der an zwei Stellen an das Metallatom M koordiniert ist. Im Sinne der vorliegenden Anmeldung wird der Begriff "zweizähnig" synonym mit dem Begriff "bidentat" verwendet.

Unter einem monodentaten Liganden ist ein Ligand zu verstehen, der an einer Stelle des Liganden mit dem Metallatom M koordiniert. Im Sinne der vorliegenden Anmeldung wird der Begriff "einzähnig" synonym mit dem Begriff "monodentat" verwendet.

In Abhängigkeit von der Koordinationszahl des eingesetzten Iridiums und der Natur und Zahl der eingesetzten Liganden L₁, L₂ und L₃ können verschiedene Isomere der entsprechenden Metallkomplexe bei gleichem Übergangsmetall Iridium und gleicher Natur und Zahl der eingesetzten Liganden vorliegen. Die vorliegende Erfindung betrifft jeweils einzelne Isomere der Übergangsmetallkomplexe der Formel (I) als auch Gemische verschiedener Isomere in jedem beliebigen Mischungsverhältnis. Im Allgemeinen können die verschiedenen Isomere der Übergangsmetallkomplexe der Formel (I) nach dem Fachmann bekannten Verfahren, zum Beispiel durch Chromatographie, Sublimation oder Kristallisation, getrennt werden.

In den Verbindungen der Formel (IIa) können o 0, 1, 2 oder 3, p 0, 1 oder 2 und n 0 bedeuten. Für den Fall, dass die Indices n, o bzw. p 0 bedeuten, sind die entsprechenden substituierbaren Positionen des Triphenylen-Gerüsts bzw. eines Derivats davon mit Wasserstoffatomen substituiert.

Bevorzugte Ausführungsformen der Reste R², R³ und R⁴ sind vorstehend genannt. Besonders bevorzugt bedeuten R², R³ und R⁴ C₁-C₄-Alkyl, zum Beispiel Methyl, Ethyl, n-Propyl, i-Propyl, n-Butyl, sec-Butyl, i-Butyl oder tert-Butyl, mit Halogen substituiertes C₁-C₄-Alkyl, bevorzugt mit F-substituiertes Alkyl, zum Beispiel CF₃, C₁-C₄-Alkoxy, zum Beispiel OMe, OEt, O*n*Pr, O*i*Pr, O*n*Bu, O*sec*Bu, O*i*Bu, O*tert*Bu, Halogen, bevorzugt F oder Pseudohalogen, bevorzugt CN.

Die Reste R², R³ und R⁴ in den Gruppen X², X³ und X⁴ weisen unabhängig voneinander die vorstehend für die Reste R², R³ und R⁴ genannten Definitionen auf.

In einer bevorzugten Ausführungsform der vorliegenden Erfindung weisen die Reste und Indices in den Triphenylenderivaten der Formel (II) die folgenden Bedeutungen auf:
- R¹: die oben genannten Bedeutungen;
- R², R³, R⁴: unabhängig voneinander C₁-C₂₀-Alkyl, C₀-C₂₀-Alkylen-C₃-C₁₈-cycloalkyl, C₀-C₂₀- Alkylen-heterocycloalkyl mit 3 bis 18 Ringatomen, C₀-C₂₀-Alkylen-C₁-C₂₀-alkoxy, C₀-C₂₀-Alkylen-C₆-C₁₈-aryloxy, C₀-C₂₀-Alkylen-C₆-C₁₈-aryl, C₀-C₂₀-Alkylen- heteroaryl mit 5 bis 18 Ringatomen, wobei die vorstehend genannten Reste mit Hydroxy, Halogen, Alkyl, Cycloalkyl, Heterocycloalkyl, Aryl, Heteroaryl oder Amino, substituiert oder unsubstituiert sein können; Pseudohalogen oder Halo- gen, wobei weiter bevorzugte Reste R², R³ und R⁴ vorstehend genannt sind;
- o: 0 bis 3, wobei die Reste R³ bei o > 1 gleich oder verschieden sein können;
- p: 0 bis 2, wobei die Reste R⁴ bei p > 1 gleich oder verschieden sein können;
- n: 0;
- X²: CH;
- X³: CH oder CR³;
- X⁴: unabhängig voneinander CH oder CR⁴.

Ganz besonders bevorzugt sind in den Verbindungen der Formel (IIa) X², X³ und X⁴ CH. In einer weiteren ganz besonders bevorzugten Ausführungsform sind die Indices n, o und p 0.

Bei dem Metallatom M in den metallorganischen Komplexen der allgemeinen Formel (I) handelt es sich um Ir in jeder für Iridium möglichen Oxidationsstufe. Insbesondere ganz besonders bevorzugt ist das Metallatom M Ir(III).

Beispiele für geeignete Reste R¹ sind: 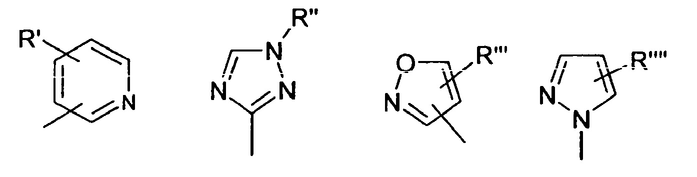 wobei R', R", R'" und R"" die bezüglich R^{a} genannten Bedeutungen aufweisen können.

Des Weiteren können die vorstehend genannten Reste R¹ zusätzlich anellierte Gruppen tragen, wobei Benzanellierungen bevorzugt sind. Ein Beispiel für einen geeigneten benzanellierten Rest R¹ ist:

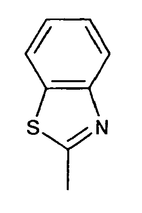

Beipiele für besonders bevorzugte Triphenylenderivate der Formel IIa sind im Folgenden genannt, wobei das Triphenylengerüst gegebenenfalls weitere Substituenten tragen kann und/oder eine oder mehrere CH-Gruppen des Triphenylen-Grundgerüsts durch N ersetzt sein können:

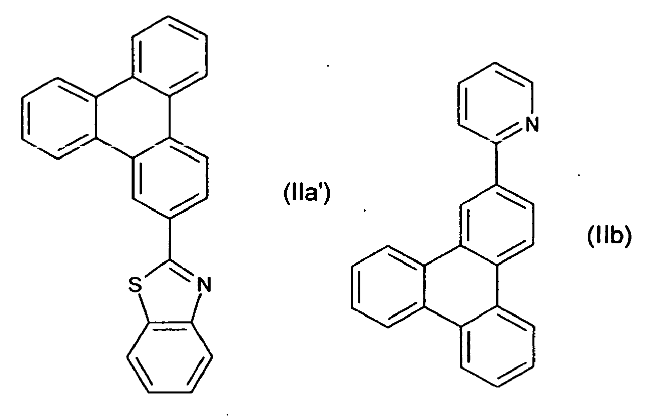

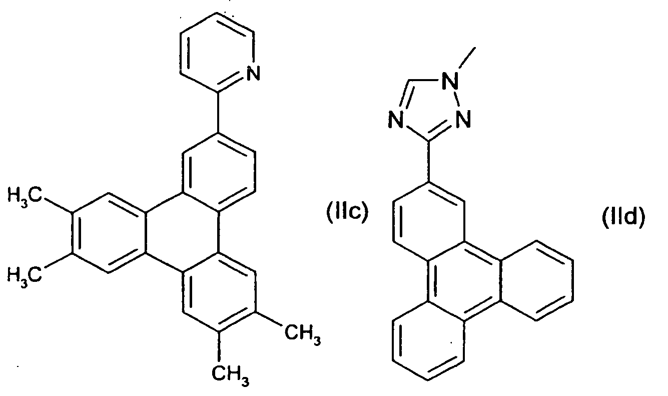

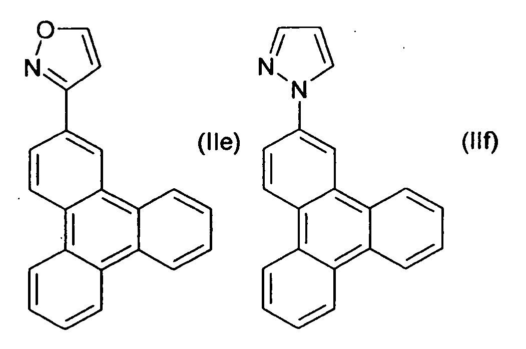

Es handelt es sich bei dem Liganden L₁ in den metallorganischen Komplexen der allgemeinen Formel (I) um einen monoanionischen bidentaten Liganden.

Die metallorganischen Komplexe der allgemeinen Formel (I) enthalten einen, zwei oder drei Liganden L₁, wobei in dem Fall, wenn mehr als ein Ligand L₁ in den metallorganischen Komplexen der Formel (I) vorliegt, die Liganden L₁ gleich oder verschieden sein können. In einer Ausführungsform der vorliegenden Erfindung enthält der metallorganischen Komplex der allgemeinen Formel (1) zwei Liganden L₁. Das bedeutet, dass q in den metallorganischen Komplexen der allgemeinen Formen (I) 1, 2 oder 3, bevorzugt 1 oder 2, besonders bevorzugt 2 ist, wobei die Liganden L₁ bei q > 1 gleich oder verschieden sein können.

Bei dem Liganden L₂ in den metallorganischen Komplexen der allgemeinen Formel (I) handelt es sich um einen mono- oder dianionischen Ligand, der mono- oder bidentat sein kann.

Geeignete mono- oder dianionische Liganden L₂, die mono- oder bidentat sein können, sind üblicherweise als mono- oder bidentate mono- oder dianionische Liganden eingesetzte Liganden.

Geeignete monoanionische monodentate Liganden sind zum Beispiel Halogenide, insbesondere Cl⁻ und Br⁻, Pseudohalogenide, insbesondere CN-, Cyclopentadienyl (Cp⁻), Hydrid, Alkylreste, die mit dem Metall M über eine Sigmabindung verknüpft sind, zum Beispiel CH₃, Alkylarylreste, die mit dem Metall M über eine Slgmabindung verknüpft sind, zum Beispiel Benzyl.

Geeignete monoanionische bidentate Liganden sind zum Beispiel Acetylacetonat und dessen Derivate, Picolinat, Schiffsche Basen, Aminosäuren, Arylacyl, zum Beispiel Phenylpyridin, sowie die weiteren in WO 02/15645 genannten bidentaten monoanionischen Liganden, wobei Acetylacetonat und Piccolinat bevorzugt sind.

Geeignete dianionische bidentate Liganden sind zum Beispiel Dialkoholate, Dicarbonate, Dicarboxylate, Diamide, Diimide, Dithiolate, Biscyclopentadienyle, Bisphosphonate, Bissulfonate und 3-Phenylpyrazol.

Besonders bevorzugte geeignete Liganden L₂ sind die folgenden Liganden (a) bis (f) 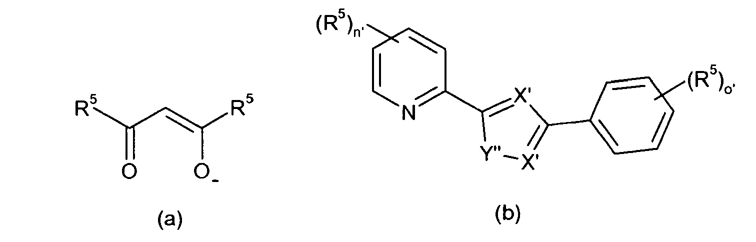 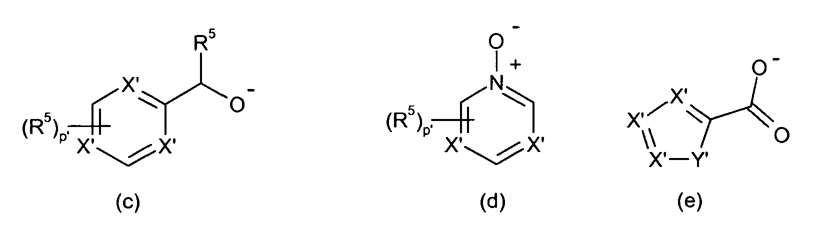 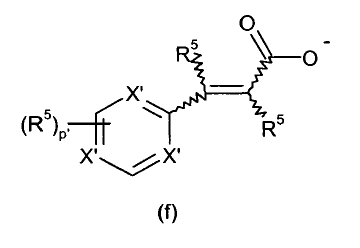 worin bedeuten:
- R⁵: in jedem der Liganden (a) bis (f) unabhängig voneinander Wasserstoff, C₁-C₂₀- Alkyl, C₀-C₂₀-Alkylen-C₃-C₁₈-cycloalkyl, C₀-C₂₀-Alkylen-heterocycloalkyl mit 3 bis 18 Ringatomen, C₀-C₂₀-Alkylen-C₁-C₂₀-alkoxy, C₀-C₂₀-Alkylen-C₆-C₁₈-aryloxy, C₀- C₂₀-Alkylen-C₆-C₁₈-aryl, C₀-C₂₀-Alkylen-heteroaryl mit 5 bis 18 Ringatomen, wo- bei die vorstehend genannten Reste mit Hydroxy, Halogen, Pseudohalogen, Al- kyl, Cycloalkyl, Heterocycloalkyl, Aryl, Heteroaryl, Amino, -C(O)R', -C(O)OR", - OC(O)R"', -OC(O)OR"", wobei R', R", R'" und R"" unabhängig voneinander Wasserstoff, Alkyl, Cycloalkyl, Heterocycloalkyl, Aryl, Heteroaryl oder Amino be- deuten, substituiert oder unsubstituiert sein können; Hydroxy, Halogen, Pseudo- halogen, Phosphonat, Phosphat, Phosphin, Phosphinoxid, Phosphoryl, Sulfonyl, Sulfonat, Sulfat, Sulfon, Amino, Polyether, Silyl-C₁-C₂₀-alkyl, Silyl-C₀-C₂₀-Alkylen- C₆-C₁₈-aryl, Silyl-C₁-C₂₀-Alkylen-C₁-C₂₀-alkoxy, -C(O)R', -C(O)OR", -OC(O)R"', - OC(O)OR"", wobei R', R", R'" und R"" unabhängig voneinander Wasserstoff, Alkyl, Cycloalkyl, Heterocycloalkyl, Aryl, Heteroaryl oder Amino bedeuten; bevorzugt bedeutet R⁵ Wasserstoff, C₁-C₆-Alkyl, C₀-C₄-Alkylen-C₃-C₈-Cycloalkyl, C₀-C₄-Alkylen-C₆-C₁₈-Aryl;
- X': unabhängig voneinander CR⁵ oder N;
- Y': C(R⁵)₂, NR⁵, O oder S;
- Y": N⁻;
- n': 1, 2, 3 oder 4;
- o': 1, 2, 3, 4 oder 5,
- p': 1 oder 2.

Ganz besonders bevorzugt ist der Ligand L₂ ausgewählt aus der Gruppe bestehend aus β-Diketonaten wie Acetylacetonat und dessen Derivaten, Picolinat, Aminosäureanionen und monoanionischen bidentaten Liganden der allgemeinen Formel (b), wobei alle Gruppen X' in der Formel (b) besonders bevorzugt N bedeuten.

Die geschlängelte Linie in den Liganden der allgemeinen Formel (f) bedeutet, dass alle möglichen cis/trans-lsomere durch die allgemeine Formel (f) umfasst sind.

Die erfindungsgemäßen metallorganischen Komplexe der Formel (I) können 0, 1, 2, 3 oder 4 Liganden L₂ aufweisen. Dabei können die Liganden L₂ bei dem Vorliegen von mehr als einem Liganden L₂ in den metallorganischen Komplexen der Formel (I) gleich oder verschieden sein. Bevorzugt weisen die metallorganischen Komplexen der allgemeinen Formel (I) einen oder zwei Liganden L₂ auf. Das bedeutet, dass r in den metallorganischen Komplexen der Formel (I) 0 bis 4, bevorzugt 1 oder 2 bedeutet.

Die metallorganischen Komplexe der Formel (I) können des Weiteren gegebenenfalls einen oder mehrere neutrale mono- oder bidentate Liganden L₃ aufweisen.

Geeignete neutrale mono- oder bidentate Liganden L₃ sind bevorzugt ausgewählt aus der Gruppe bestehend aus Phosphinen, sowohl Mono- als auch Bisphosphinen; Phosphonaten, sowohl Mono- als auch Bisphosphonaten, und Derivaten davon; Arsenaten, sowohl Mono- als auch Bisarsenaten, und Derivaten davon; Phosphiten, sowohl Mono- als auch Bisphosphiten; CO; Pyridinen, sowohl Mono- als auch Bispyridinen; Nitrilen, Dinitrilen, Allyl, Diiminen, nicht konjugierten Dienen und konjugierten Dienen, die einen π-Komplex mit dem Metall M bilden. Besonders bevorzugte neutrale mono- oder bidentate Liganden L₃ sind ausgewählt aus der Gruppe bestehend aus Phosphinen, sowohl Mono- als auch Bisphosphinen, bevorzugt Trialkyl-, Triaryl- oder Alkylarylphosphinen, besonders bevorzugt PAr₃, wobei Ar ein substituierter oder unsubstituierter Arylrest ist und die drei Arylreste in PAr₃ gleich oder verschieden sein können, besonders bevorzugt PPh₃, PEt₃, PnBu₃, PEt₂Ph, PMe₂Ph, P*n*Bu₂Ph; Phosphonaten und Derivaten davon, Arsenaten und Derivaten davon, Phosphiten, CO; Pyridinen, sowohl Mono- als auch Bispyridinen, wobei die Pyridine mit Alkyl- oder Arylgruppen substituiert sein können; Nitrilen und Dienen, die einen π-Komplex mit dem Metall M bilden, bevorzugt η⁴-1,4-Dibenzyl-1,3-butadien, η⁴-2,4-Hexadien, η⁴-3-Methyl-1,3-pentadien, η⁴-1,4-Dibutyl-1,3-butadien, η⁴-1,4-bis(Trimethylsilyl)-1,3-butadien und η²-oder η⁴-Cyclooctadien (je 1,3 und je 1,5), besonders bevorzugt 1,4-Diphenyl-1,3-Butadien, 1-Phenyl-1,3-pentadien, 2,4-Hexadien, Butadien, η²-Cycloocten, η⁴-1,3-Cyclooctadien und η⁴-1,5-Cyclooctadien. Ganz besonders bevorzugte neutrale monodentate Liganden sind ausgewählt aus der Gruppe bestehend aus PPh₃, P(OPh)₃, CO; Pyridin, Nitrilen und deren Derivaten. Geeignete neutrale mono- bzw. bidentate Liganden sind bevorzugt 1,4-Diphenyl-1,3-butadien, 1-Phenyl-1,3-pentadien, 2,4-Hexadien, η⁴-Cyclooctadien und η²-Cyclooctadien (je 1,3 und je 1,5).

Die metallorganischen Komplexe der Formel (I) können 0, 1, 2, 3 oder 4 neutrale mono- oder bidentate Liganden L₃ aufweisen. Falls mehr als 1 Ligand L₃ in den Übergangsmetallkomplexen der Formel (I) vorliegt, können die Liganden L₃ gleich oder verschieden sein. In einer bevorzugten Ausführungsform enthält der metallorganische Komplex der allgemeinen Formel (1) 0 Liganden L₃. Das bedeutet, dass s in den metallorganischen Komplexen der allgemeinen Formel (I) 0 bis 4, bevorzugt 0 bedeutet.

In einer besonders bevorzugten Ausführungsform betrifft die vorliegende Erfindung metallorganische Komplexe gemäß Formel (I), worin bedeuten
- M: Ir(III);
- L₂: ein monoanionischer bidentater Ligand, wobei bevorzugte monoanionische bi- dentate Liganden bereits vorstehend genannt sind,
- q: 1 oder 2, bevorzugt 2;
- r: 1 oder 2;
- s: 0;
- L₁: ein monoanionischer bidentater Ligand, abgeleitet von einem Triphenylenderivat der Formel (IIa), wobei bevorzugte Triphenylenderivate vorstehend genannt sind,
wobei die Summe aus q + r = 3 bedeutet.

Besonders bevorzugte metallorganische Komplexe der Formel (I) sind metallorganische Komplexe der folgenden Formeln (Ia), (Ib) und (Ic) und (Id), (Ie) und (If)

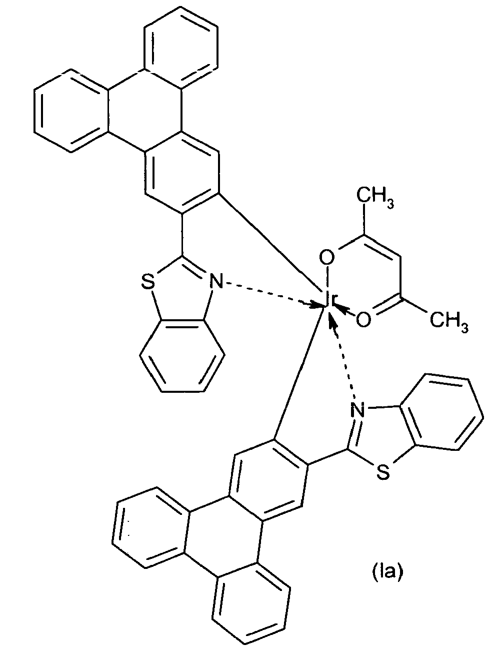

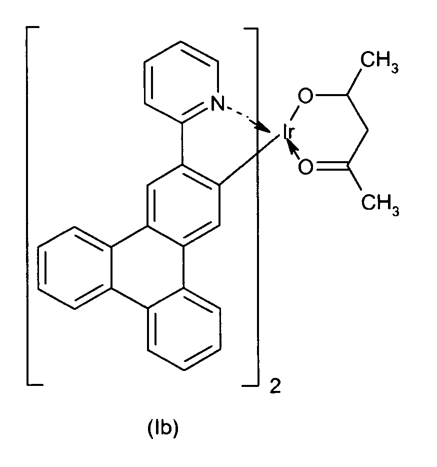

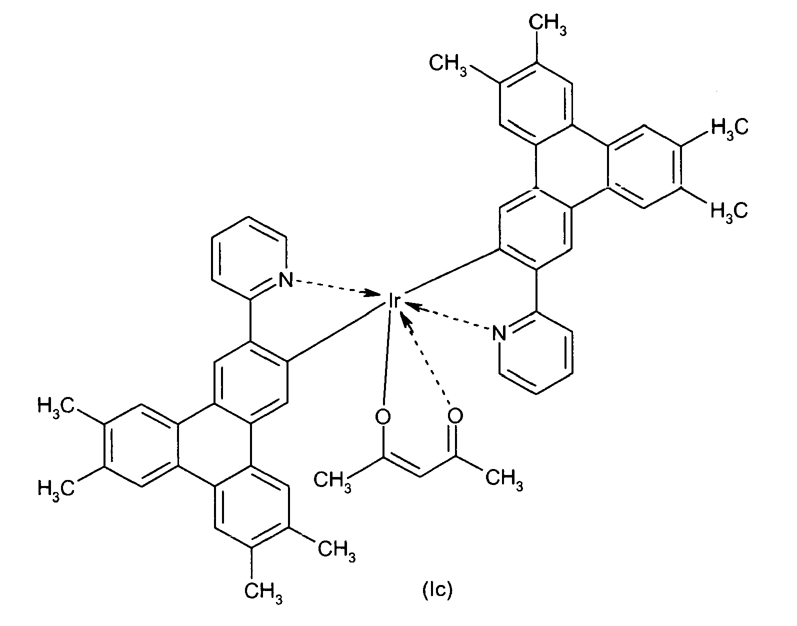

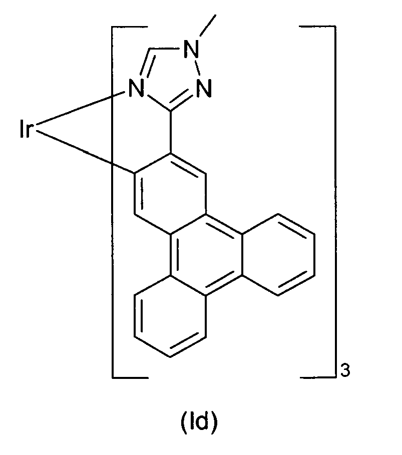

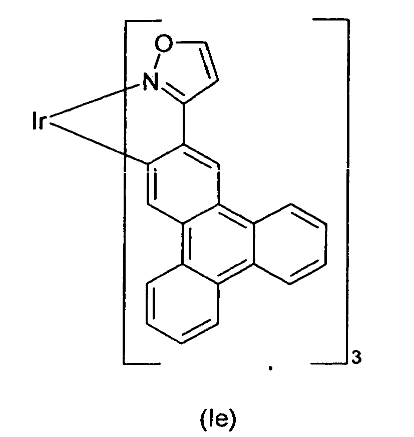

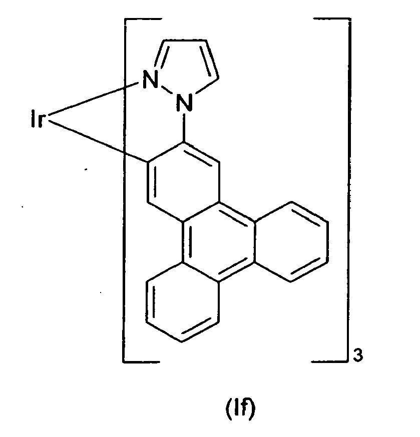

Die Herstellung der erfindungsgemäßen metallorganischen Komplexe (I) kann nach allen dem Fachmann bekannten Verfahren erfolgen.

In einer bevorzugten Ausführungsform erfolgt die Herstellung durch
(a) Umsetzung von Iridiumsalzen oder Iridiumkomplexen, die gegebenenfalls einen oder mehrere Liganden L₃ enthalten, mit einem ersten Liganden L₁ oder L₂ zu Iridiumkomplexen, die entweder einen oder mehreren Liganden L₁ oder einen oder mehrere Liganden L₂, gegebenenfalls neben einem oder mehreren Liganden L₃, tragen;
(b) Umsetzung der in Schritt (a) erhaltenen Iridiumkomplexe mit einem zweiten Liganden L₁, wenn der in Schritt (a) erhaltene Iridiumkomplex einen oder mehrere Liganden L₂ enthält, bzw. mit einem Liganden L₂, wenn der in Schritt (a) erhaltene lridiumkomplex einen oder mehrere Liganden L₁ enthält, wobei ein metallorganischer Komplex der Formel (I) erhalten wird, wobei Schritt (b) in dem Fall, dass der metallorganische Komplex der Formel (I) keine Liganden L₂ enthält, d.h. wenn r in dem metallorganischen Komplex der Formel (I) 0 bedeutet, entfällt.

Die Reaktionsbedingungen zur Herstellung von metallorganischen Komplexen ausgehend von geeigneten Liganden sind dem Fachmann bekannt.

Die erfindungsgemäßen metallorganischen Komplexe der Formel (I) sind als Emittermaterialien insbesondere für den Einsatz in OLEDs geeignet. Im Allgemeinen werden die Emittermaterialien gemeinsam mit einem oder mehreren geeigneten Matrix-Materialien eingesetzt. Ein Vorteil der erfindungsgemäßen Übergangsmetallkomplexe ist, dass diese aufgrund ihrer Struktur in hohen Konzentrationen in OLEDs, insbesondere in der Licht-emittierenden Schicht, eingesetzt werden können, ohne dass die Bildung von Dimeren und damit ein Quenchen der Lumineszenz auftritt. Dadurch können OLEDs mit hoher Lumineszenz-Effizienz und hoher Lebensdauer der Licht-emittierenden Schicht bereitgestellt werden.

Üblicherweise liegen einer oder mehrere metallorganische Komplexe der Formel (I) in der Licht-emittierenden Schicht eines OLEDs vor, bevorzugt gemeinsam mit einem oder mehreren Matrix-Materialien. Die Konzentration der metallorganischen Komplexe der Formel (I) in den Matrix-Materialien beträgt im Allgemeinen > 0 bis ≤ 100 Gew.-%, bevorzugt ≥ 5 bis ≤ 50 Gew.-%, besonders bevorzugt ≥ 10 bis ≤ 30 Gew.-% und ganz besonders bevorzugt ≥ 11 bis ≤ 25 25 Gew.-%, bezogen auf die Licht-emittierende Schicht. Das Matrix-Material oder die Matrix-Materialien liegen entsprechend bevorzugt in einer Konzentration von 0 bis < 100 Gew.-%, bevorzugt von ≥ 50 bis ≤ 95 Gew.-%, besonders bevorzugt von ≥ 70 bis ≤ 90 Gew.-%, ganz besonders bevorzugt von ≥ 75 bis ≤ 89 Gew.-% vor.

Geeignete Matrix-Materialien sind dem Fachmann bekannt. Beispiele für geeignete Matrix-Materialien sind z.B. in Organic Light-Emitting Materials and Devices (Optical Science and Engineering Series), Ed.: Z. Li, H. Meng, CRC Press Inc., 2006 publiziert.

Ein weiterer Gegenstand der vorliegenden Anmeldung ist die Verwendung der erfindungsgemäßen metallorganischen Komplexe der Formel (I) oder der erfindungsgemäßen Mischungen enthaltend mindestens einen metallorganischen Komplex der Formel (I) in organischen Leuchtdioden. Bevorzugt werden die metallorganischen Komplexe der Formel (I) in der Licht-emittierenden Schicht von organischen Leuchtdioden eingesetzt.

Ein weiterer Gegenstand der vorliegenden Erfindung ist die Verwendung der erfindungsgemäßen metallorganischen Komplexe der Formel (I) als Emittermaterialien.

OLEDs sowie der Aufbau geeigneter OLEDs sind dem Fachmann bekannt.

Ein bevorzugtes Verfahren zur Herstellung von erfindungsgemäßen Triphenylenderivaten, worin R¹ ein 2-Pyridyl-Rest ist, ist nachstehend beispielhaft gezeigt. 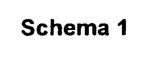 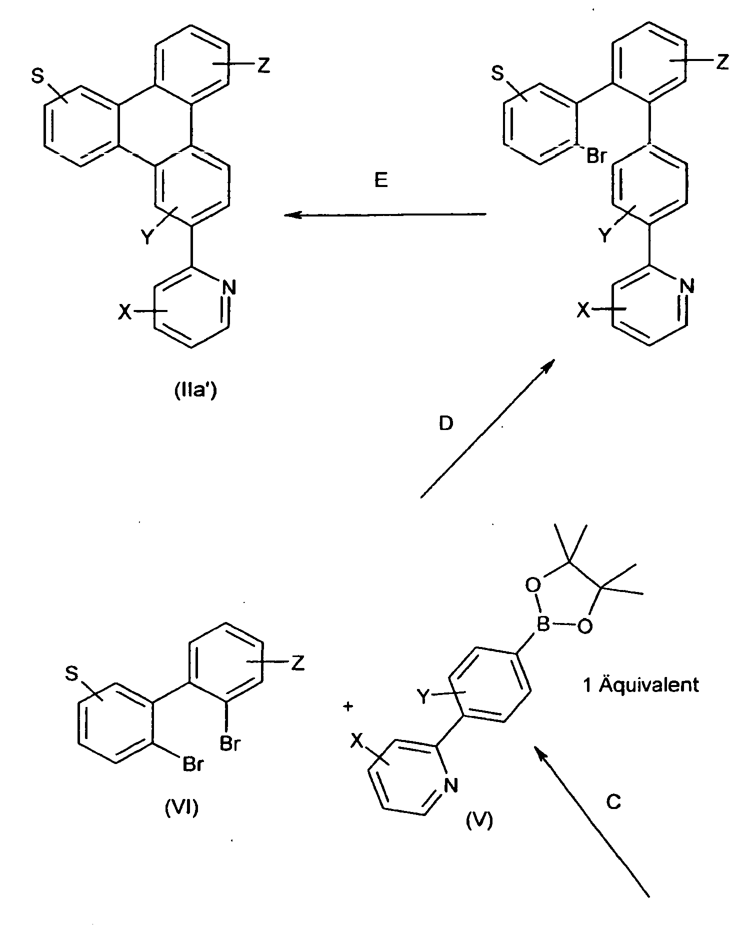 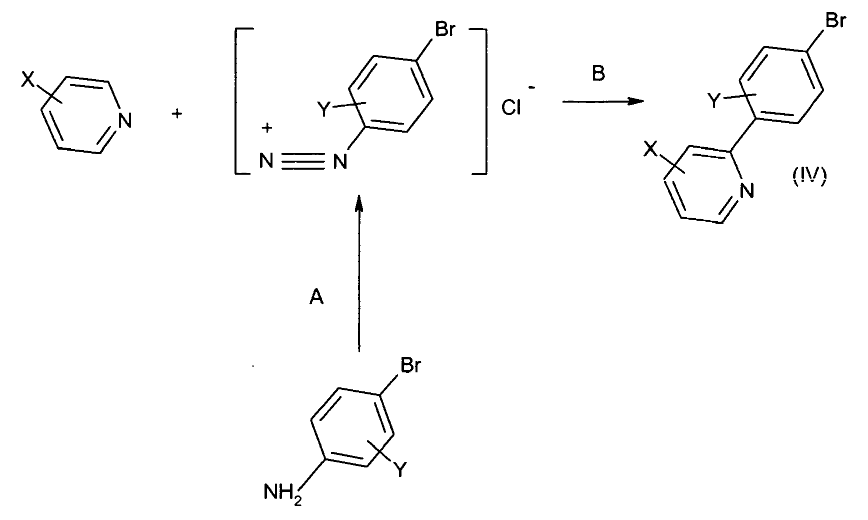 X, Y, Z und S stellen einen oder mehrere Substituenten dar, wobei geeignete Substituenten Y, Z und S den Substituenten (R²)ₙ, (R³)ₒ und (R⁴)ₚ entsprechen, die vorstehend definiert sind. Die Gruppe X entspricht den Substituenten des Restes R¹, wobei bevorzugte Substituenten R^{a} und R^{b}, die vorstehend definiert sind, sind. Geeignete Substituenten X, Y, Z und S sind z.B. Me, tBu, CF₃, F und OMe.

In den Schritten A, B und C in Schema 1 erfolgt die Herstellung einer geeigneten Arylboronsäure oder eines geeigneten Arylboronsäurederivats (V). Dabei wird zunächst eine mit einer Gruppe Y (im vorliegenden Fall Br) funktionalisierte aromatische Verbindung der Formel (IV) hergestellt. In dem vorliegenden Schema, worin R¹ Pyridin bedeutet, erfolgt die Herstellung der Verbindung der Formel (IV) durch Azokupplung der entsprechenden mit Halogen (im vorliegenden Fall Br) funktionalisierten Arylgruppe mit Pyridin. Die erhaltene Verbindung der Formel (IV) (im vorliegenden Schema 2-(4-Bromophenyl)pyridin) wird, bevorzugt durch Palladium-katalysierte Umsetzung, zu der entsprechenden Arylboronsäure oder dem entsprechenden Arylboronsäurederivat (V) umgesetzt. Geeignete Umsetzungen sind dem Fachmann bekannt. Im vorliegenden Schema erfolgt die Umsetzung von 2-(4-Bromophenyl)pyridin mit bis(Pinacolato)diboron in Anwesenheit von Pd(dba)₃ und Tricyclohexylphosphin in katalytischen Mengen in Anwesenheit einer Base, KOAc.

Die erhaltene Arylboronsäure bzw. das erhaltene Arylboronsäurederivat der Formel (V) wird in Schritt D (Schritt (ii) des erfindungsgemäßen Verfahrens) mit einem mit zwei Gruppen z funktionalisierten Biphenylderivat der Formel (VI) umgesetzt. Bei den Gruppen z handelt es sich um Halogen oder OTf, im vorliegenden Fall in Schema 1 um Br. Die Umsetzung erfolgt Palladium-katalysiert. Als Palladium-Katalysator wird in Schritt D in dem vorliegenden Schema 1 bevorzugt Pd(PPh)₄ in Anwesenheit von einer Base, Na₂CO₃, eingesetzt.

Zur Herstellung des gewünschten Triphenylenderivats der Formel (IIa') erfolgt in Schritt E in Schema 1 (Schritt (iii) des erfindungsgemäßen Verfahrens) eine Palladium-katalysierte intramolekulare Cyclisierung. Als Palladium-Katalysator wird in dem vorliegenden Schema 1 dabei Pd(OAc)₂ in katalytischen Mengen in Anwesenheit einer Base, K₂CO₃, eingesetzt.

Die in Schritt (iii) durchgeführte Palladium-katalysierte intramolekulare Cyclisierung zur Herstellung der gewünschten Triphenylenderivate der Formel (IIa') war bisher im Stand der Technik nicht bekannt. Es wurde gefunden, dass auf diese Weise die direkte Synthese von kondensierten aromatischen Systemen (Triphenylenderivaten) möglich ist.

Ein weiterer Weg zur Herstellung der erfindungsgemäßen Triphenylenderivate der Formel (IIa') ist ausgehend von 2-Triphenylencarbonsäuren möglich, wie in dem allgemeinen Schema 2 dargestellt ist:

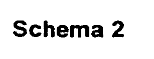

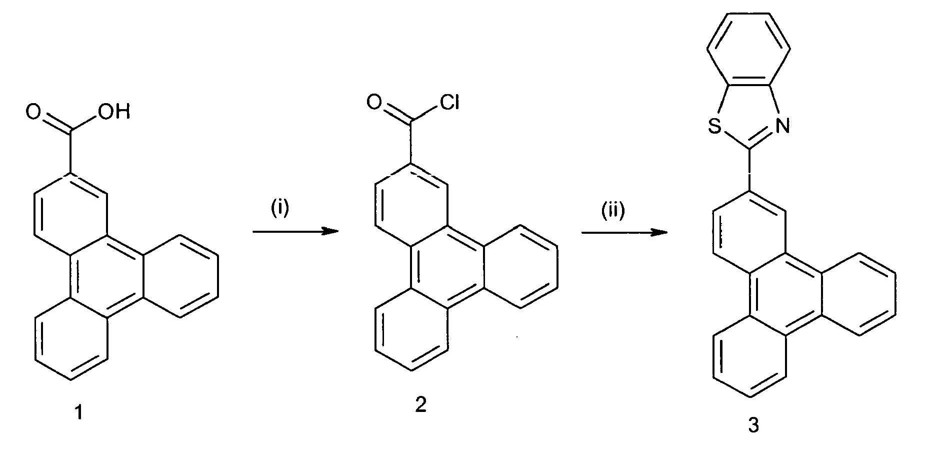

Dabei wird in Schritt (i) 2-Triphenylencarbonsäure gemäß dem Fachmann bekannten Verfahren zu dem entsprechenden Säurechlorid umgesetzt. Die Umsetzung kann mit jedem dem Fachmann bekannten Chlorierungsmittel, z. B. mit Thionylchlorid, erfolgen.

Anschließend wird in Schritt (ii) das erhaltene Säurechlorid zum Beispiel mit O-Aminothiophenol umgesetzt, wobei ein erfindungsgemäßes Triphenylenderivat der Formel (IIa) erhalten wird.

Das Schema 2 ist lediglich beispielhaft. Das Triphenylengerüst kann weitere Substituenten tragen bzw. einige der in dem Triphenylengerüst enthaltenen Kohlenstoffatome können durch Stickstoffatome ersetzt sein.

Die eingesetzten Triphenylencarbonsäuren können nach dem Fachmann bekannten Verfahren hergestellt werden.

Ein weiterer Weg zur Herstellung der erfindungsgemäßen Triphenylenderivate über 2-Triphenylencarbonsäure ist in Schema 3 dargestellt. In Schema 3 ist beispielhaft ein Verfahren zur Herstellung von erfindungsgemäßen Triphenylenderivaten der Formeln (Id) und (Ie) dargestellt. Ein Weg zur Herstellung der 2-Triphenylencarbonsäure ist ebenfalls in Schema 3 gezeigt:

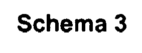

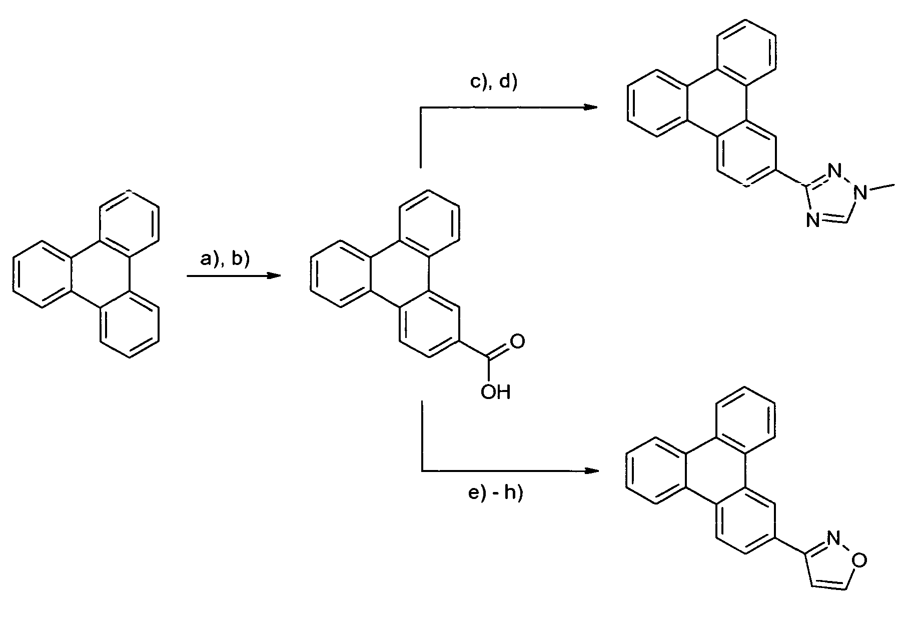

Geeignete Reaktionsbedingungen sind analogen Reaktionen in der Literatur zu entnehmen. Geeignete Literatur bezüglich der einzelnen in Schema 3 genannten Schritte ist nachfolgend aufgeführt. Besonders bevorzugte Reaktionsbedingungen sind im nachfolgenden Beispielteil genannt.
a) und b) analog Veröffentlichung für Dihydropyren: D. M. Connor, S. D. Scott, D. M. Collard, Chr. L. Liotta, D. A. Schiraldi, J. Org. Chem. 1999, 64, 6888 - 6890.
c) analog Veröffentlichung über 3-Methyl-4-nitrobenzoesäure: D. J. Sall, A. E. Arfesten, J. A. Bastian, M. L. Denney, C. S. Harms, J. Med. Chem. 1997, 40, 2843 - 2857.
d) analog Veröffentlichung über 3-(1-Methyl-1,2,4-triazol-3-yl)-azabicyclo[2.2.2]octane: H. J. Wadsworth, S. M. Jenkins, B. S. Orlek, F. Cassidy, M. S. G. Clark, F. Brown, G. J. Riley, D. Graves, J. Hawkins, Chr. B. Naylor, J. Med. Chem. 1992, 35, 1280 - 1290.
e) und f) analog Veröffentlichung für lodbenzoesäure: S. E. Gibson et al. Chem. Eur. J. 2005, 11, 69 - 80.
g) analog Veröffentlichung für Benzaldehyd Oxim: P. Aschwanden et al. Org. Lett. 2005, 7, 5741 - 5742.
h) analog Veröffentlichung für 3-substituierte Isoxazole: A. Baranski, Pol. J. Chem. 1982, 56, 1585 - 1589 bzw. R. G. Micetich, Can. J. Chem 1970, 48, 467 - 476 bzw. S. - R. Sheng, X.-L. Liu, Q. Xu, C.-S. Song, Synthesis 2006, 14, 2293 - 2296.

Das Schema 3 ist lediglich beispielhaft. Das Triphenylengerüst kann weitere Substituenten tragen bzw. einige der in dem Triphenylengerüst enthaltenen Kohlenstoffatome können durch Stickstoffatome ersetzt sein.

Ein weiterer Weg zur Herstellung der erfindungsgemäßen Triphenylenderivate ausgehend von einem Triphenylengerüst kann über eine Bromierung von Triphenylen analog zu dem Fachmann bekannten Verfahren erfolgen. In Schema 4 ist dieser Weg beispielhaft zur Herstellung des Triphenylenderivats der Formel (If) gezeigt:

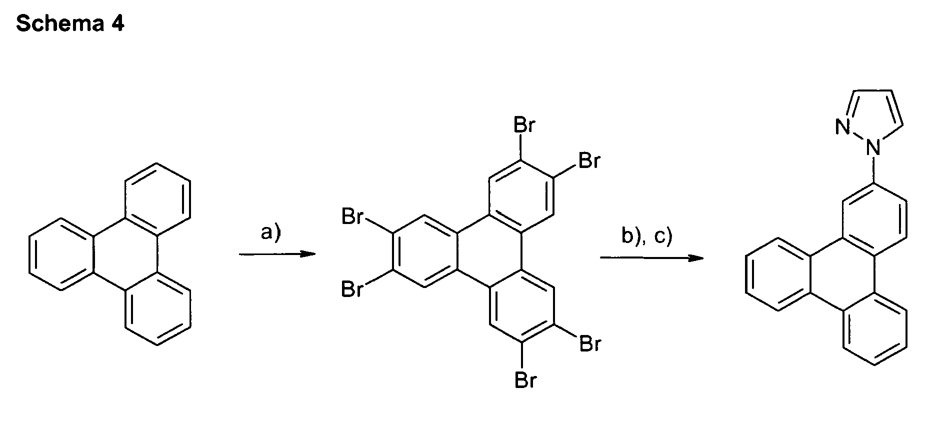

Geeignete Reaktionsbedingungen sind analogen Reaktionen in der Literatur zu entnehmen. Geeignete Literatur bezüglich der einzelnen in Schema 4 genannten Schritte ist nachfolgend aufgeführt. Besonders bevorzugte Reaktionsbedingungen sind im nachfolgenden Beispielteil genannt.
a) analog Veröffentlichung von R. Breslow, Ronald, B. Jaun, Bernhard, R. Q. Kluttz,C.-z. Xia, Tetrahedron 1982, 38, 863-867.
b) analog Veröffentlichung für Phenylpyrazol: J. C. Antilla, J. M. Baskin, T. E. Barder, S. L. Buchwald, J. Org. Chem. 2004, 69, 5578 - 5587.
c₁) analog Veröffentlichung für Dibromchlorbenzol: K. Menzel, L. Dimichele, P. Mills, D. E. Frantz, T. D. Nelson, M. H. Kress, Syn. Lett. 2006, 12, 1948 - 1952.
c₂) analog Veröffentlichung für Tetrabromaromaten: G. Dorman, J. D. Olszewski, G. D. Prestwich, Y. Hong, D. G. Ahern, David G, J. Org. Chem. 1995, 60, 2292-2297.
c₃) analog Veröffentlichung zur Debromierung von Aromaten: S. Arai, M. Oku, T. Ishida, T. Shioiri; Tetrahedron Lett. 1999, 40, 6785 - 6790.

Das Schema 4 ist lediglich beispielhaft. Das Triphenylengerüst kann weitere Substituenten tragen bzw. einige der in dem Triphenylengerüst enthaltenen Kohlenstoffatome können durch Stickstoffatome ersetzt sein. Insbesondere können gemäß Schema 4 auch die entsprechenden erfindungsgemäßen Triphenylenderivate der Formeln (Id) und (Ie) hergestellt werden.

Des Weiteren können die erfindungsgemäßen Triphenylenderivate durch Arin-Kupplung erhalten werden. Ein allgemeines Schema 5 ist im Folgenden angegeben. In Schema 5a ist die Herstellung durch Arin-Kupplung am Beispiel der Herstellung der Verbindungen der Formeln (Id), (Ie) und (If) gezeigt:

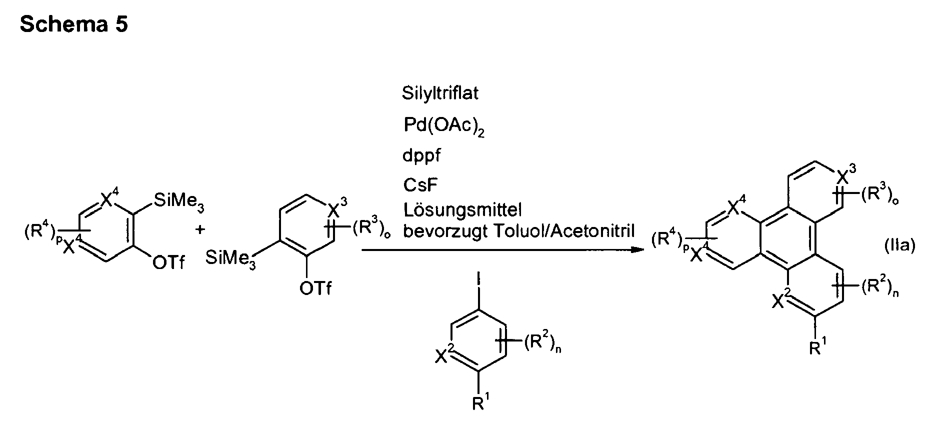

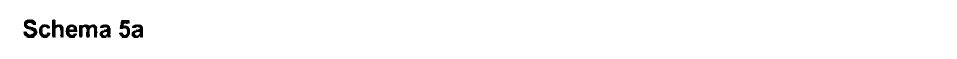

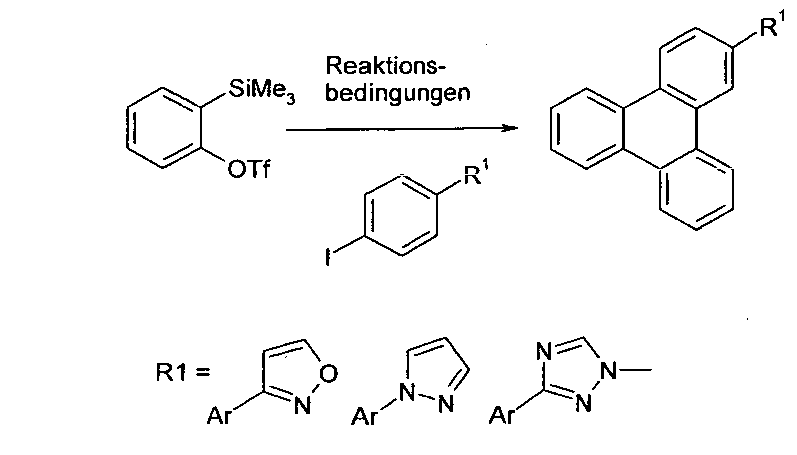

Die Reaktionsbedingungen sind analog zur Herstellung von Methyltriphenylen, wie z.B. in Z. Liu, R. Larock, J. Org. Chem. 2007, 72, 223 - 232 offenbart. Besonders bevorzugte Reaktionsbedingungen sind im nachfolgenden Beispielteil genannt.

Die nachfolgenden Beispiele erläutern die Erfindung Zusätzlich.

### Beispiele

Herstellung von Triphenylenderivaten der Formel (IIa') gemäß Schema 1:

### 1. Herstellung von 2-(4-(4,4,5,5-Tetramethyl-1,3,2-dioxaborolan)pyridin

0,39 g (0,4 mmol) Pd(dba)₃ und 0,28 g (1 mmol) Tricyclohexylphosphin werden in 10 ml trockenem Dioxan unter Stickstoffatmosphäre suspendiert. Die erhaltene Mischung wird 30 Minuten bei Raumtemperatur gerührt. Anschließend werden nach und nach 5,3 g (15 mmol) bis (Pinacolato)diboron, 2,1 g (21 mmol) KOAc und 3,3 g (14 mmol) 2-(4-Bromophenyl)pyridin zugegeben. Die Reaktionsmischung wird für 20 Stunden unter Rückfluss gekocht, abgekühlt und mit 10 ml Wasser bei Raumtemperatur behandelt. Das Produkt wird mit Dichlormethan extrahiert. Das Lösungsmittel wird im Vakuum entfernt und das erhaltene Rohprodukt wird über eine kurze Kieselgelsäule gereinigt. Nach Reinigung über die Kieselgelsäule (Dichlormethan/Hexan, 3 : 1) werden 82% des gewünschten Produktes erhalten.
¹H-NMR(CDCl₃): δ = 1,37(s, 12H), 7,22-7,26(m, 1H), 7,72-7,80(m, 2H), 7,92(J = 8,4 Hz, 2H), 8,02(J = 8,2Hz, 2H), 8,71 (J = 4,9 Hz, 1H).

### 2. Herstellung eines mit zwei Br-Resten substituierten Diphenylderivats der Formel (VI)

Die Herstellung von substituierten Dibromiden erfolgt ausgehend von o-Dibrombenzolen. Ein typisches Verfahren umfasst die Reaktionssequenz Lithiierung/Kupplung. Ein allgemeines Verfahren ist in der folgenden Literaturstelle offenbart: H. S. M. Kabir et al., J. Chem. Suc., Perkin Trans. 1, 2001, 159-165 (Synthese von 2, 2'-Dibrom-4,4',5, 5'-Tetramethylbiphenyl).

### 3. Kupplung des Arylboronsäurederivats (V) mit dem mit zwei Br-Resten funktionalisierten Biphenylderivat der Formel (VI)

6,4 mmol des Dibromids (VI) und 6,4 mmol des Arylboronsäurederivats (V) (2-(4-(4,4,5,5-Tetramethyl-1,3,2-Dioxaborolan)pyrridin) werden in 25 ml Toluol gelöst. Es werden 0,4 mmol [Pd(PPh₃)₄] und 10 ml einer 4N-Lösung von Na₂CO₃ zugegeben und die Mischung wird unter Stickstoff bis zum Rückfluss erhitzt. Die Reaktionsmischung wird für 6 Stunden unter Rückfluss erhitzt, gekühlt, die gebildeten Phasen werden getrennt und die Wasserphase wird mit Dichlormethan extrahiert und getrocknet. Das Lösungsmittel wird im Vakuum entfernt und das Rohprodukt wird über eine kurze Kieselgelsäule gereinigt. Nach Reinigung durch Säulenchromatographie (Dichlormethan/Hexan, 2:1) wird das gewünschte o-Terphenylderivat in 40- bis 50%-iger Ausbeute erhalten.

### 4. Intramolekulare Cyclisierung zu dem Triphenylenderivat der Formel (IIa')

2,87 mmol des o-Terphenylderivats, 0,14 mmol Pd(OAc)₂ und 5,7 mmol K₂CO₃ werden in 7 ml DMA bei 135°C für 24 Stunden unter Stickstoffatmosphäre erhitzt. Die Reaktionsmischung wird abgekühlt, mit 5 ml Wasser behandelt und mit Dichlormethan extrahiert. Die organische Phase wird getrocknet, das Lösungsmittel im Vakuum entfernt und über eine kurze Kieselgelsäule gereinigt (Dichlormethan). Nach Säulenchromatographie (Dichlormethan/Hexan, 2:1) wird das gewünschte Triphenylenderivat (IIa') in 35 bis 40% Ausbeute erhalten.

### 5. Herstellung der erfindungsgemäßen Triphenylenderivate der Formel (IIa') gemäß Schema 2

0,8 g (2,9 mmol) 2-Triphenylencarbonsäure (hergestellt gemäß dem Fachmann bekannten Verfahren) werden in 15 ml Chloroform suspendiert. Anschließend werden **4** ml Thionylchlorid zugegeben, und die Reaktionsmischung wird unter Stickstoffatmosphäre zum Rückfluss gebracht. Nach dreistündigem Rühren unter Rückfluss wird die klare Lösung im Vakuum evakuiert und der feste Rückstand aus Hexan/Chloroform (10/1) umkristallisiert. Das erhaltene Säurechlorid **2** wird in 10 ml trockenem 1-Methylpyrrolidinon gelöst und 0,32 ml (2,9 mmol) o-Aminothiophenol werden zugegeben. Die Reaktionsmischung wird bei 100°C für drei Stunden gerührt. Nach Abkühlen wird die Lösung in kaltes Wasser gegeben und die Mischung auf einen pH-Wert von 8 bis 9 mit 7 N-wässrigem Ammoniak eingestellt. Der gebildete Niederschlag wird filtriert, mit Wasser gewaschen und über eine kurze Kieselgelsäule (Dichlormethan) gereinigt, wobei 0,82 g (78%) des gewünschten Triphenylenderivats (IIa') 3 erhalten werden.
Smp: 230 bis 231 °C.

### 6. Herstellung eines erfindungsgemäßen Übergangsmetallkomplexes

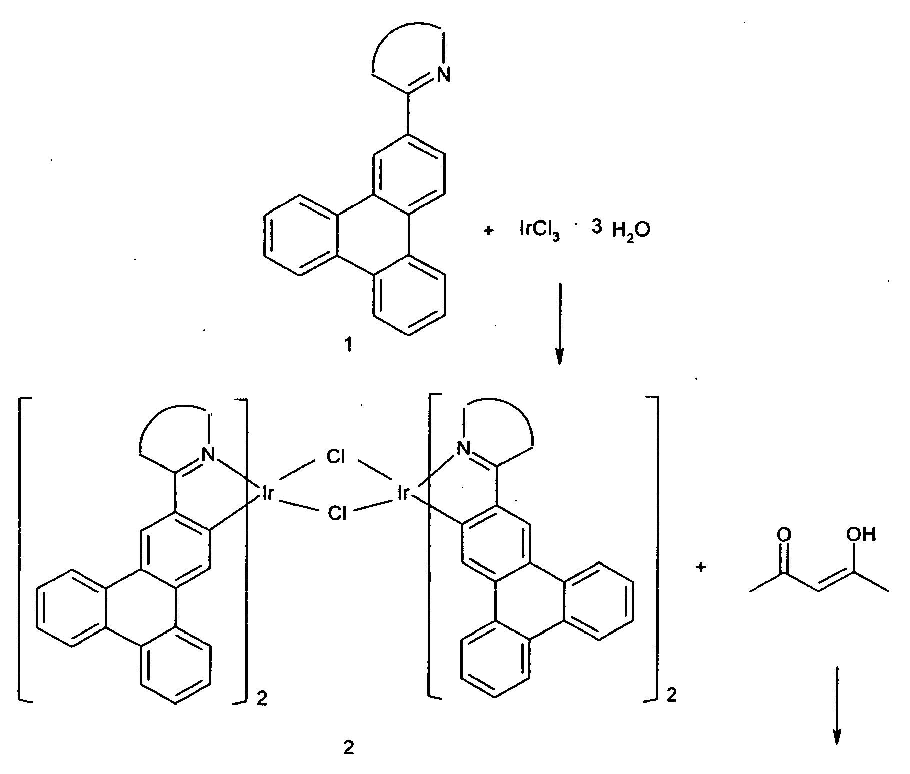

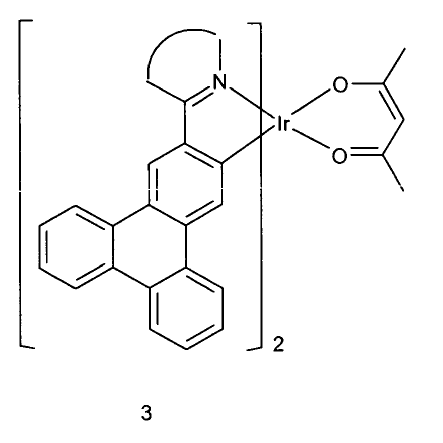

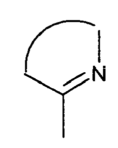

heterocyclischer Rest R¹, wobei geeignete heterocyclische Reste R¹ vorstehend genannt sind

### Allgemeine Vorschrift

0,83 mmol des Liganden **1** werden in 30 ml 2-Ethoxyethanol in Stickstoffatmosphäre unter Erwärmen suspendiert. 0,38 mmol IrCl₃ . 3H₂O werden zugegeben und die erhaltene Suspension wird zum Rückfluss gebracht. In 30 Minuten erscheint ein farbiger Niederschlag. Die Reaktionsmischung wird für 24 Stunden unter Rückfluss gehalten und anschließend auf Raumtemperatur abgekühlt. Der Niederschlag wird durch Sedimentation in einer Zentrifuge gesammelt und intensiv mit Methanol (6x15ml) gewaschen. Nach Trocknen im Hochvakuum unter Heizen (T = 80°C) wird das Dichloroverbrückte Dimer **2** in 80 bis 90%-iger Ausbeute erhalten. Diese Komplexe sind in üblichen organischen Lösungsmitteln schwer löslich und werden ohne weitere Reinigung weiter verwendet.

0,16 mmol des Dichloro-verbrückten Dimers 2 werden in 10 ml 2-Ethoxyethanol unter Stickstoffatmosphäre suspendiert. 0,4 mmol Acetylaceton und 85 bis 90 mg Na₂CO₃ werden zugegeben und die Reaktionsmischung wird bei 100°C für fünf Stunden gerührt. Die erhaltene Suspension wird auf Raumtemperatur abgekühlt, mit Wasser verdünnt und der farbige Niederschlag wird durch Sedimentation in einer Zentrifuge gesammelt, intensiv mit Wasser/Methanol (4/1,6x15ml) gewaschen und im Hochvakuum unter Erhitzen (T = 100°C) getrocknet. Nach Reinigung durch Säurenchromatographie wird der Komplex **3** als farbiger Feststoff in 70- bis 80%-iger Ausbeute erhalten.

Gemäß der vorstehend genannten Vorschrift werden die Ir-Komplexe Ia und Ib erhalten:

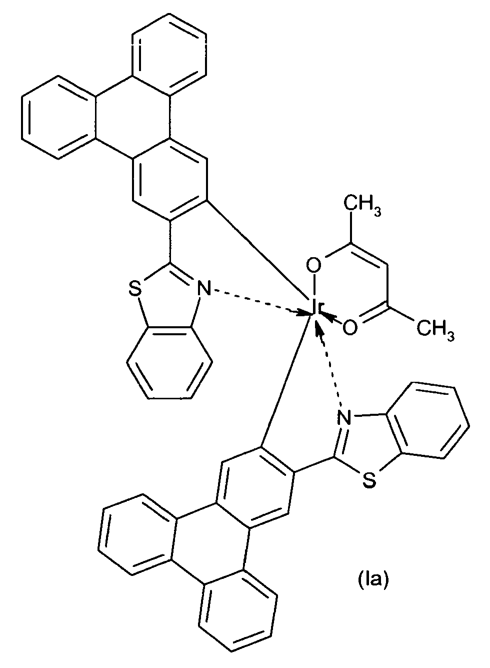

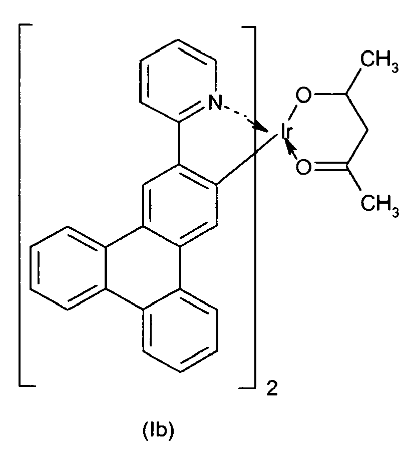

### 7. Einsatz des erfindungsgemäßen Übergangsmetallkomplexes Ia und Ib in einer OLED

Der Diodenaufbau ist wie folgt:
- Glas-Substrat mit 120 nm Indium-ZinnOxid
- Lochinjektionslage (PEDOT-PSS) 200 nm
- Lochtransportlage aus MTDATA, undotiert; 100 nm
- Emissionslage aus alpha-NPD, mit 9% (per Gewicht) Iridiumkomplex Ia (Beispiel 1) oder Ib (Beispiel 2) dotiert; 250 nm
- Lage zur Lochblockade und zum Elektronentransport, TPBI, 100 nm
- Elektroneninjektionslage (Lithiumfluorid) 450nm
- Kathode aus Aluminium, 70 nm

Die Ergebnisse sind in der folgenden Tabelle zusammengefaßt, wobei in Beisüiel 1 der Ir-Komplex Ia eingesetzt wurde und in Beispiel 2 der Ir-Komplex Ib:

| Effizienz | Beispiel 1 | Beispiel 2 |
|---|---|---|
| Emissionsmaximum | 589 nm | 541 nm |
| Effizienz | 12 Im/W at 1000 nit | 32 Im/W at 1000 nit |

### 8. Herstellung der erfindungsgemäßen Triphenylenderivate der Formel (IIa) gemäß Schema 3

### 8.1 Herstellung von 2-Triphenylencarbonsäure

### Schritt a)

Triphenylen (1 Äquivalent) wird bei 0°C mit 2,1 Äquivalenten AlCl₃ und 21,0 Äquivalenten CH₃COCl in CH₂Cl₂ umgesetzt. Nach 3 stüdigem Rühren bei Raumtemp. wurde das Umsetzungsprodukt (Acetyltriphenylen) in 97%iger Ausbeute erhalten, welches in Schritt b) eingesetzt wird.

### Schritt b)

Das in Schritt a) erhaltene Umsetzungsprodukt wird mit 2,2 Äquivalenten I₂ (bezogen auf die Roh-Ausbeute an Acetyltriphenylen) in Pyridin Lösungsmittel bei Raumtemperatur versetzt. Darauf wird 45 min bei Rückfluß gehalten, worauf eine weitere Portion I₂ (1,0 Äquivalent) zugegeben wird. Nach einer weiteren Stunde Rückfluß wird NaOH, EtOH und Wasser zugegeben und die Reaktionsmischung 2 h zum Rückfluss erhitzt. Man erhält 2-Triphenylencarbonsäure in 76%iger Ausbeute (bezogen auf Roh-Ausbeute Acetyltriphenylen bzw. 74% bezogen auf Triphenylen).

### 8.2 Herstellung eines Triphenylenderivats der Formel IId

### Schritt c)

1 Äquivalent 2-Triphenylencarbonsäure aus Schritt b) wird mit PCl₅ (2,1 Äquivalente) und 1,2 Äquivalenten p-Toluolsulfonsäureamid in Xylol umgesetzt, wobei die Temperatur bei der Umsetzung 17 h bei 120°C gehalten wird. Bei 190°C werden Lösungsmittel und Reagenzien abdestilliert. Nach Abkühlen auf 5°C wird Pyridin zugegeben und wäßrig aufgearbeitet. Das in 52%iger Ausbeute erhaltene Umsetzungsprodukt wird in Schritt d) eingesetzt.

### Schritt d)

Das gemäß Schritt d) erhaltene Umsetzungsprodukt (1 Äquivalent) wird bei 0°C mit gasförmigem HCl in Ethanol versetzt. Es wird 24 Stunden bei Raumtemperatur nachgerührt. Das Lösungsmittel wird fast vollständig entfernt. Anschließend werden Ethanol als Lösungsmittel, 1,3 Äquivalente MeNHNH₂ und 2,5 Äquivalente NEt₃ zugegeben.

Es wird 24 Stunden bei Raumtemperatur gerührt. Bei 0°C wird das Reaktionsvolumen auf ¼ reduziert, HCO₂ zugegeben und das EtOH vollständig abgezogen. Anschließend wird nach Zugabe von weiterer HCO₂H bei Raumtemperatur und 2-stündigem Refluxieren das Triphenylenderivat der Formel IId erhalten.

### 8.3 Herstellung eines Triphenylenderivats der Formel IIe

### Schritt e)

1 Äquivalent 2-Triphenylencarbonsäure aus Schritt b) wird mit 2,0 Äquivalenten BH₃ THF in THF bei Raumtemperatur für 16 Stunden gerührt. Das Reaktionsprodukt wird nach der wäßrigen Aufarbeitung in Schritt f) weiter umgesetzt.

### Schritt f)

Das Reaktionsprodukt aus Schritt e) wird mit MnO₂ (25,0 Äquivalente, bezogen auf 2-Triphenylencarbonsäure) in CHCl₃ als Lösungsmittel für 3 Tage unter Rückfluss umgesetzt. Das Reaktionsprodukt wird nach der Filtraton über Celite in Schritt g) weiter umgesetzt.

### Schritt g)

Das Reaktionsprodukt aus Schritt f) wird mit 3,3 Äquivalenten (bezogen auf 2-Triphenylencarbonsäure) H₂NOH HCl und 9,0 Äquivalenten NaOH in EtOH für 1 Stunde bei Raumtemperatur und für 30 min unter Rückfluß gerührt. Das in 80-90%iger Ausbeute erhaltene Reaktionsprodukt (2-Triphenylenaldoxim) wird nach der wäßrigen Aufarbeitung in Schritt h) weiter umgesetzt.

### Schritt h)

Das Reaktionsprodukt aus Schritt g) wird mit 1,0 Äquivalenten (bezogen auf 2-Triphenylenaldoxim) NCS in CHCl₃ 30 min gerührt. Darauf wird mit Vinylbromid (1,0 Äquivalente, bezogen auf 2-Triphenylenaldoxim) versetzt und tropfenweise NEt₃ (1,1 Äquivalente) zugegeben, wobei nach 12stüdigem Rühren bei Raumtemperatur und wäßriger Aufarbeitung das Triphenylenderivat der Formel IIe erhalten wird. Alternativ zu Vinylbromid kann Vinylacetat oder Phenylvinylselenid eingesetzt werden, wobei bei Verwendung von Vinylacetat ein zusätzlicher Refluxierungsschritt vor der Aufarbeitung hinzukommt, während der Einsatz von Vinylselenid die Zugabe von 30%iger H₂O₂ bei 0°C vor der Aufarbeitung bedingt (in diesem Fall enfällt der Refluxierungsschritt).

### 9. Herstellung der erfindungsgemäßen Triphenylenderivate der Formel (IIa) gemäß Schema 4

### Schritt a)

Triphenylen (1 Äquivalent) wird mit 8 Äquivalenten Br₂ in Anwesenheit katalytischer Mengen Eisen in Nitrobenzol bromiert, wobei 80% bromiertes Triphenylenderivat erhalten werden.

### Schritt b)

Das bromierte Triphenylenderivat (1 Äquivalent) wird anschließend mit 5-10 mol% Cul, 20 mol-% Amin (N,N-Dimethylcyclohexan-1,2-diamin oder Phenantrolin), 1,0 Äquivalent Pyrazol und 2,1 Äquivalenten Base (K₂CO₃, CsCO₃ oder NaOtBu) bei 110°C in Toluol für 24 Stunden gerührt.

### Schritt c)

Das in Schritt b) erhaltene Reaktionsprodukt wird anschließend entweder in Schritt c1), in Schritt c2) oder in Schritt c3) zu dem Triphenylenderivat der Formel IIf umgesetzt: c₁) iPrMgCl·LiCl; HCl; c₂) H₂, NEt₃, Pd(OH)₂/C; c₃) HCO₂H NEt₃, P(oTol)₃, Pd(OA_{C})₂, DMF, 50°C, 24 h.

### 10. Herstellung der erfindungsgemäßen Triphenyclenderivate der Formel (IIa) gemäß Schema 5a

1-Trifluormethansulfonato-2-trimethylsilylbenzol (3 Äquivalent) wird mit 1 Äquivalent des entsprechenden lodoaromaten umgesetzt (siehe Schema 5a), in Anwesenheit von 5 mol% Pd(OAc)₂, 5 mol-% dppf und 4 Äquivalenten CsF in Toluol/Acetonitril, wobei die gewünschen Liganden der Formeln IId), IIe) bzw. IIf) erhalten werden.

## Patentansprüche

1. Metallorganischer Komplex der allgemeinen Formel (I)
M[L₁]_{q}[L₂]ᵣ[L₃]ₛ (I)
worin bedeuten
M Ir;
L₁ monoanionischer bidentater Ligand, basierend auf einem Triphenylenderi- vat der Formel (IIa) 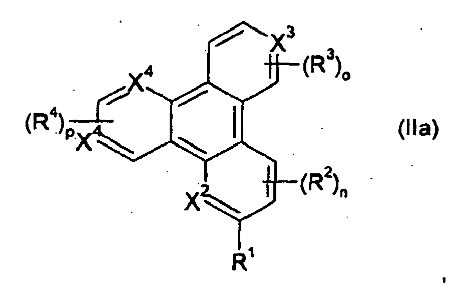 worin bedeuten:
R¹ Rest der Formel 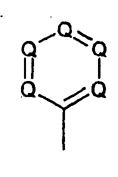 wobei Q jeweils unabhängig voneinander CR^{a} oder N bedeutet, wobei mindestens eine Gruppe Q in ortho-Stellung zu der Bindungsstelle N bedeutet und R^{a} unabhängig voneinander Wasserstoff, Alkyl, Cycloalkyl, Heterocycloalkyl, Aryl, Heteroaryl, Amino oder eine Gruppe mit Donor- oder Akzeptorwirkung bedeutet;
oder ein Rest der Formeln 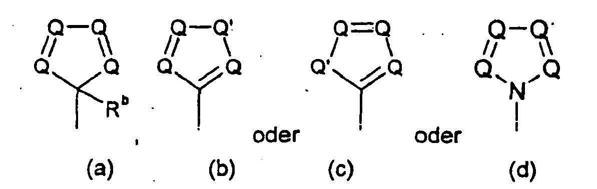 wobei Q jeweils unabhängig voneinander CR^{a} oder N bedeutet, wobei mindestens eine Gruppe Q in ortho-Stellung zu der Bindungsstelle N bedeutet, und Q' CR^{a}₂, O, S oder NR^{c} bedeutet und R^{a}, R^{b} und R^{c} unabhängig voneinander Wasserstoff, Alkyl, Cycloalkyl, Heterocycloalkyl, Aryl, Heteroaryl, Amino, CF₃, CN, Alkoxy oder F bedeuten,
R², R³, R⁴ unabhängig voneinander C₁-C₂₀-Alkyl, C₀-C₂₀-Alkylen-C₃-C₁₈-cycloalkyl, C₀-C₂₀-Alkylen-heterocycloalkyl mit 3 bis 18 Ringatomen, C₀-C₂₀-Alkylen- C₁-C₂₀-alkoxy, C₀-C₂₀-Alkylen-C₆-C₁₈-aryloxy, C₀-C₂₀-Alkylen-C₆-C₁₈-aryl, C₀-C₂₀-Alkylen-heteroaryl mit 5 bis 18 Ringatomen, wobei die vorstehend genannten Reste mit Hydroxy, Halogen, Pseudohalogen, Alkyl, Cycloal- kyl, Heterocycloalkyl, Aryl, Heteroaryl, Amino, -C(O)R', -C(O)OR", OC(O)R"', -OC(O)OR"", wobei R', R", R"' und R"" unabhängig vonein- ander Wasserstoff, Alkyl, Cycloalkyl, Heterocycloalkyl, Aryl, Heteroaryl oder Amino bedeuten, substituiert oder unsubstituiert sein können; Hydroxy, Halogen, Pseudohalogen, Phosphonat, Phosphat, Phosphin, Phosphinoxid, Phosphoryl, Sulfonyl, Sulfonat, Sulfat, Amino, Polyether, Silyl-C₁-C₂₀-alkyl, Silyl-C₀-C₂₀-Alkylen-C₆-C₁₈-aryl, Silyl-C₀-C₂₀-Alkylen- C₁-C₂₀-alkoxy, -C(O)R', -C(O)OR", -OC(O)R"', -OC(O)OR"", wobei R', R", R"' und R"" unabhängig voneinander Wasserstoff, Alkyl, Cycloalkyl, Heterocycloalkyl, Aryl, Heteroaryl oder Amino bedeuten;
o 0 bis 3, wobei die Reste R³ bei o > 1 gleich oder verschieden sein können;
p 0 bis 2, wobei die Reste R⁴ bei p > 1 gleich oder verschieden sein können;
n 0;
X² CH ;
X³ N, CH oder CR³;
X⁴ unabhängig voneinander N, CH oder CR⁴;
L₂ mono- oder dianionischer Ligand, der mono- oder bidentat sein kann;
L₃ neutraler mono- oder bidentater Ligand;
q Zahl der Liganden L₁, wobei q 1, 2 oder 3 ist und die Liganden L₁ bei q > 1 gleich oder verschieden sein können;
r Zahl der Liganden L₂, wobei r 0 bis 4 ist und die Liganden L₂ bei r > 1 gleich oder verschieden sein können;
s Zahl der Liganden L₃, wobei s 0 bis 4 ist und die Liganden L3 bei s > 1 gleich oder verschieden sein können;
wobei die Summe q + r + s von der Oxidationsstufe und Koordinationszahl des eingesetzten Metalls und von der Zähnigkeit der Liganden L₁, L₂ und L₃ sowie von der Ladung der Liganden L₁ und L₂ abhängig ist.

2. Metallorganischer Komplex nach Anspruch 1, **dadurch gekennzeichnet, dass** der Ligand L₁ eine Triplettenergie von mindestens 16000 cm⁻¹, bevorzugt von 16000 cm⁻¹ bis 19500 cm⁻¹ aufweist.

3. Metallorganischer Komplex nach Anspruch 1 oder 2, **dadurch gekennzeichnet, dass** die Reste und Indizes in dem Triphenylenderivat der Formel (II) die folgenden Bedeutungen aufweisen
R², R³, R⁴ unabhängig voneinander C₁-C₂₀-Alkyl, C₀-C₂₀-Alkylen-C₃-C₁₀-cycloalkyl, C₀-C₂₀-Alkylen-heterocycloalkyl mit 3 bis 18 Ringatomen, C₀-C₂₀-Alkylen- C₁-C₂₀-alkoxy, C₀-C₂₀-Alkylen-C₆-C₁₈-aryloxy, C₀-C₂₀-Alkylen-C₉-C₁₈-aryl, C₀-C₂₀-Alkylen-heteroaryl mit 5 bis 18 Ringatomen, wobei die vorstehend genannten Reste mit Hydroxy, Halogen, Alkyl, Cycloalkyl, Heterocycloal- kyl, Aryl, Heteroaryl oder Amino, substituiert oder unsubstituiert sein kön- nen; Pseudohalogen oder Halogen;
ο 0 bis 3, wobei die Reste R³ bei o > 1 gleich oder verschieden sein können;
p 0 bis 2, wobei die Reste R⁴ bei p > 1 gleich oder verschieden sein können;
n 0;
X² CH ;
X³ CH oder CR³;
X⁴ unabhängig voneinander CH oder CR⁴.

4. Metallorganischer Komplex nach einem der Ansprüche 1 bis 3, **dadurch gekennzeichnet, dass** das Metallatom M Ir(III) ist.

5. Metallorganischer Komplex nach einem der Ansprüche 1 bis 4, **dadurch gekennzeichnet, dass**
M Ir(III);
L₂ ein monoanionischer bidentater Ligand;
q 1 oder 2;
r 1 oder 2;
s 0
ist, und die Summe q + r = 3 ist.

6. Metallorganischer Komplex nach einem der Ansprüche 1 bis 5, **dadurch gekennzeichnet, dass** L₂ ausgewählt ist aus der Gruppe bestehend aus β-Diketonaten wie Acetylacteonat und dessen Derivate, Picolinat, Aminosäureanionen und monoanionischen bidentaten Liganden der allgemeinen Formel (b) 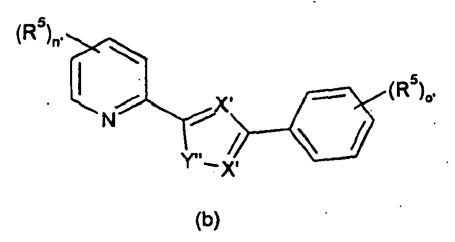 worin bedeuten
R⁵ Wasserstoff, C₁-C₆-Alkyl, C₀-C₄₋Alkylen-C₃-C₈-Cycloalkyl, C₀-C₄-Alkylen-C₆- C₁₈-Aryl;
X' N;
Y" N⁻;
n' 1, 2, 3 oder 4; und
o' 1, 2, 3, 4 oder 5.

7. Verfahren zur Herstellung von metallorganischen Komplexen nach einem der Ansprüche 1 bis 6 durch
(a) Umsetzung von Iridiumsalzen oder Iridiumkomplexen, die gegebenenfalls einen oder mehrere Liganden L₃ enthalten, mit einem ersten Liganden L₁ oder L₂ zu Iridiumkomplexen, die entweder einen oder mehrere Liganden L₁ oder einen oder mehrere Liganden L₂, gegebenenfalls neben einem oder mehreren Liganden L₃, tragen;
(b) Umsetzung der in Schritt (a) erhaltenen Iridiumkomplex einem zweiten Liganden L₁, wenn der in Schritt (a) erhaltene Iridiumkomplex einen oder mehrere Liganden L₂ enthält bzw. mit einem zweiten Liganden L₂, wenn der in Schritt (a) erhaltene Inridiumkomplex einen oder mehrere Liganden L₁ enthält, wobei ein metallorganischer Komplex der Formel (I) erhalten wird, wobei Schritt (b) in dem Fall, dass der metallorganische Komplex der Formel (I) keine Liganden L₂ enthält, d.h. wenn r in dem metallorganischen Komplex der Formel (I) 0 bedeutet, entfällt.

8. Mischung enthaltend mindestens ein Matrixmaterial und mindestens .einen metallorganischen Komplex nach einem der Ansprüche 1 bis 6 oder hergestellt nach Anspruch 7.

9. Verwendung von metallorganischen Komplexen nach einem der Ansprüche 1 bis 6 oder hergestellt nach Anspruch 7 oder Mischungen nach Anspruch 8 in organischen Leuchtdioden.

10. Verwendung nach Anspruch 8 oder 9, **dadurch gekennzeichnet, dass** die metallorganischen Komplexe als Emittermaterialien eingesetzt werden.

## Claims

1. An organometallic complex of the general formula (I)
M[L₁]_{q}[L₂]ᵣ[L₃]ₛ (I)
in which
M is Ir;
L₁ is a monoanionic bidentate ligand based on a triphenylene derivative of the formula (IIa) 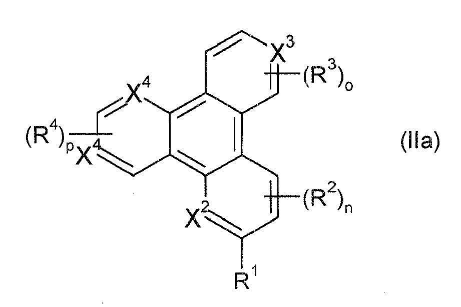 in which
R¹ is a radical of the formula 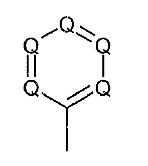 where Q is in each case independently CR^{a} or N, where at least one Q group in the ortho-position to the bonding site is N and R^{a} is independently hydrogen, alkyl, cycloalkyl, heterocycloalkyl, aryl, heteroaryl, amino or a group having donor or acceptor action;
or a radical of the formula 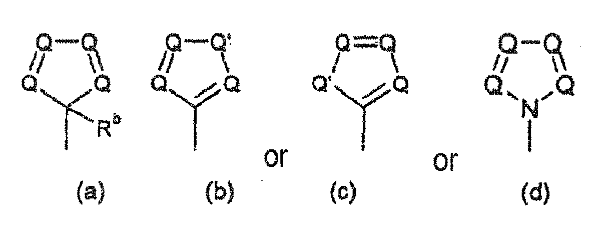 where Q is in each case independently CR^{a} or N, where at least one Q group in the ortho-position to the bonding site is N, and Q' is CR^{a}₂, O, S or NR^{c} and R^{a}, R^{b} and R^{c} are each independently hydrogen, alkyl, cycloalkyl, heterocycloalkyl, aryl, heteroaryl, amino, CF₃, CN, alkoxy or F,
R², R³ R⁴ are each independently C₁-C₂₀-alkyl, C₀-C₂₀- alkylene-C₃-C₁₈-cycloalkyl, C₀-C₂₀- alkyleneheterocycloalkyl having from 3 to 18 ring atoms, C₀-C₂₀-alkylene-C₁-C₂₀-alkoxy, C₀-C₂₀- alkylene-C₆-C₁₈-aryloxy, C₀-C₂₀-alkylene-C₆-C₁₈- aryl, C₀-C₂₀-alkyleneheteroaryl having from 5 to 18 ring atoms, where the aforementioned radicals may be substituted by hydroxyl, halogen, pseudohalogen, alkyl, cycloalkyl, heterocycloalkyl, aryl, heteroaryl, amino, -C(O)R', -C(O)OR", -OC(O)R"', -OC(O)OR"", where R', R", R"' and R"" are each independently hydrogen, alkyl, cycloalkyl, heterocycloalkyl, aryl, heteroaryl or amino, or be unsubstituted; hydroxyl, halogen, pseudohalogen, phosphonate, phosphate, phosphine, phosphine oxide, phosphoryl, sulfonyl, sulfonate, sulfate, amino, polyether, silyl-C₁-C₂₀-alkyl, silyl-C₀-C₂₀-alkylene-C₆-C₁₈- aryl, silyl-C₀-C₂₀-alkylene-C₁-C₂₀-alkoxy, - C(O)R', -C(O)OR", -OC(O)R"", -OC(O)OR"", where R' , R" , R"' and R"" are each independently hydrogen, alkyl, cycloalkyl, heterocycloalkyl, aryl, heteroaryl or amino;
o is from 0 to 3, where the R³ radicals, where o > 1, may be the same or different;
p is from 0 to 2, where the R⁴ radicals, when p > 1, may be the same or different;
n is 0;
X² is CH;
X³ is N, CH or CR³;
X⁴ are each independently N, CH or CR⁴;
L₂ is a mono- or dianionic ligand which may be mono- or bidentate;
L₃ is an uncharged mono- or bidentate ligand;
q is the number of ligands L₁, where q is 1, 2 or 3 and the ligands L₁, when q > 1, may be the same or different;
r is the number of ligands L₂, where r is from 0 to 4 and the ligands L₂, when r > 1, may be the same or different;
s is the number of ligands L₃, where s is from 0 to 4 and the ligands L₃, when s > 1, may be the same or different;
where the sum of q + r + s depends on the oxidation stage and coordination number of the metal used and on the density of the ligands L₁, L₂ and L₃ and also on the charge of the ligands L₁ and L₂.

2. The organometallic complex according to claim 1, wherein ligand L₁ has a triplet energy of at least 16 000 cm⁻¹, preferably from 16 000 cm⁻¹ to 19 500 cm⁻¹.

3. The organometallic complex according to claim 1 or 2, wherein the radicals and indices in the triphenylene derivative of the formula (II) are each defined as follows
R², R³, R⁴ are each independently C₁-C₂₀-alkyl, C₀-C₂₀- alkylene-C₃-C₁₈-cycloalkyl, C₀-C₂₀- alkyleneheterocycloalkyl having from 3 to 18 ring atoms, C₀-C₂₀-alkylene-C₁-C₂₀ alkoxy, C₀-C₂₀- alkylene-C₆-C₁₈-aryloxy, C₀-C₂₀-alkylene-C₆-C₁₈- aryl, C₀-C₂₀-alkyleneheteroaryl having from 5 to 18 ring atoms, where the aforementioned radicals may be substituted by hydroxy halogen, alkyl, cycloalkyl, heterocycloalkyl, aryl, heteroaryl or amino, or be unsubstituted; pseudohalogen or halogen;
o is from 0 to 3, where the R³ radicals, when o > 1, may be the same or different;
p is from 0 to 2, where the R⁴ radicals, when p > 1, may be the same or different;
n is 0;
X² is CH;
X³ is CH or CR³;
X⁴ are each independently CH or CR⁴.

4. The organometallic complex according to any of claims 1 to 3, wherein the metal atom M is Ir (III) .

5. The organometallic complex according to any of claims 1 to 4, wherein
M is Ir(III);
L₂ is a monoanionic bidentate ligand;
q is 1 or 2;
r is 1 or 2;
s is 0
and the sum of q + r = 3.

6. The organometallic complex according to any of claims 1 to 5, wherein L₂ is selected from the group consisting of β-diketonates such as acetylacetonate and derivatives thereof, picolinate, amino acid anions and monoanionic bidentate ligands of the general formula (b) 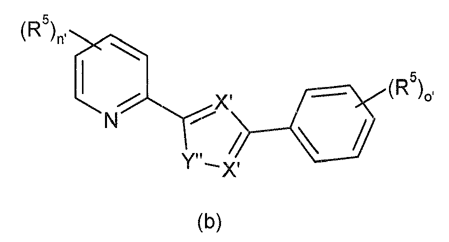 in which
R⁵ is hydrogen, C₁-C₆-alkyl, C₀-C₄-alkylene-C₃-C₈- cycloalkyl, C₀-C₄-alkylene-C₆-C₁₈-aryl;
X' is N;
V is N ;
n' is 1, 2, 3 or 4; and
o' is 1, 2, 3, 4 or 5.

7. A process for preparing organometallic complexes according to any of claims 1 to 6 by
(a) reacting iridium salts or iridium complexes which optionally comprise one or more ligands L₃ with a first ligand L₁ or L₂ to give iridium complexes which bear either one or more ligands L₁ or one or more ligands L₂, if appropriate in addition to one or more ligands L₃;
(b) reacting the iridium complexes obtained in step (a) with a second ligand L₁ when the iridium complex obtained in step (a) comprises one or more ligands L₂, or with a second ligand L₂, when the iridium complex obtained in step (a) comprises one or more ligands L₁, to obtain an organometallic complex of the formula (I), step (b) being dispensed with in the case that the organometallic complex of the formula (I) does not comprise any ligand L₂, i.e. when r in the organometallic complex of the formula (I) is 0.

8. A mixture comprising at least one matrix material and at least one organometallic complex according to any of claims 1 to 6 or prepared according to claim 7.

9. The use of organometallic complexes according to any of claims 1 to 6 or prepared according to claim 7 or mixtures according to claim 8 in organic light-emitting diodes.

10. The use according to claim 8 or 9 wherein the organometallic complexes are used as emitter materials.

## Revendications

1. Complexe métallo-organique de formule générale (I)
M[L₁]_{q}[L₂]ᵣ[L₃]ₛ (1)
dans laquelle
M signifie Ir ;
L₁ signifie un ligand bidentate monoanionique, à base d'un dérivé de triphénylène de formule (IIa) 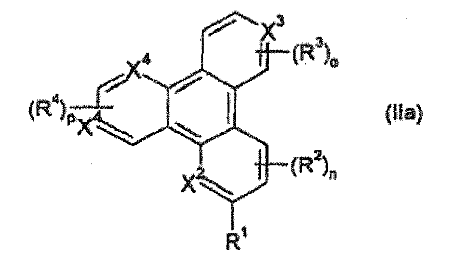 dans laquelle :
R¹ signifie un radical de formule 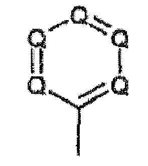 où Q signifie à chaque fois, indépendamment l'un de l'autre, CR^{a} ou N, où au moins un groupe Q en position ortho par rapport au site de liaison signifie N et R^{a} signifie, indépendamment l'un de l'autre, hydrogène, alkyle, cycloalkyle, hétérocycloalkyle, aryle, hétéroaryle, amino ou un groupe avec un effet donneur ou accepteur ;
ou un radical des formules 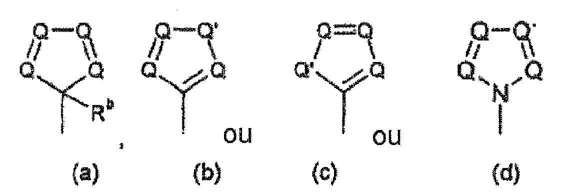 où Q signifie à chaque fois, indépendamment l'un de l'autre, CR^{a} ou N, où au moins un groupe Q en position ortho par rapport au site de liaison signifie N et Q' signifie CR^{a}₂, O, S ou NR^{c} et R^{a}, R^{b} et R^{c} signifient, indépendamment l'un de l'autre, hydrogène, alkyle, cycloalkyle, hétérocycloalkyle, aryle, hétéroaryle, amino, CF₃, CN, alcoxy ou F,
R²,R³, R⁴ signifient, indépendamment l'un de l'autre, C₁-C₂₀- alkyle, C₀-C₂₀-alkylène-C₃-C₁₈-cycloalkyle, C₀-C₂₀- alkylène-hétérocycloalkyle comprenant 3 à 18 atomes de cycle, C₀-C₂ₒ-alkylène-C₁-C₂₀-alcoxy, C₀- C₂₀-alkylène-C₆-C₁₈-aryloxy, C₀-C₂₀-alkylène-C₆-C₁₈- aryle, C₀-C₂₀-alkylène-hétéroaryle comprenant 5 à 18 atomes de cycle, où les radicaux susmentionnés peuvent être substitués par hydroxy, halogène, pseudohalogène, alkyle, cycloalkyle, hétérocycloalkyle, aryle, hétéroaryle, amino, -C(O)R', -C(O)OR", -OC(O)R''', -OC(O)OR'''', où R', R", R"' et R"" signifient, indépendamment l'un de l'autre, hydrogène, alkyle, cycloalkyle, hétérocycloalkyle, aryle, hétéroaryle ou amino, ou non substitués hydroxy, halogène, pseudohalogène, phosphonate, phosphate, phosphine, phosphinoxyde, phosphoryle, sulfonyle, sulfonate, sulfate, amino, polyéther, silyl-C₁-C₂₀-alkyle, silyl-C₀-C₂₀-alkylène-C₆-C₁₈-aryle, silyl-C₀-C₂₀- alkylène-C₁-C₂₀-alcoxy, -C(O)R', -C(O)OR'', -OC(O)R"', -OC(O)OR'''', où R', R", R"' et R"" signifient, indépendamment l'un de l'autre, hydrogène, alkyle, cycloalkyle, hétérocycloalkyle, aryle, hétéroaryle ou amino ;
O vaut 0 à 3, où les radicaux R³, lorsque O > 1, peuvent être identiques ou différents ;
p vaut 0 à 2, où les radicaux R⁴, lorsque p > 1, peuvent être identiques ou différents ; n 0 ;
X² signifie CH ;
X³ signifie N, CH ou CR³ ;
X⁴ signifient, indépendamment l'un de l'autre, N, CH ou CR⁴ ;
L₂ signifie un ligand monoanionique ou dianionique, qui peut être monodentate ou bidentate ;
L₃ signifie un ligand monodentate ou bidentate neutre
q signifie le nombre de ligands L₁, où q vaut 1, 2 ou 3 et les ligands L₁ pour q > 1 peuvent être identiques ou différents ;
r signifie le nombre de ligands L₂, où r vaut 0 à 4 et les ligands L₂ pour r > 1 peuvent être identiques ou différents ;
s signifie le nombre de ligands L₃, où s vaut 0 à 4 et les ligands L₃ pour s > 1 peuvent être identiques ou différents ;
où la somme q + r + s dépend de l'étage d'oxydation et de l'indice de coordination du métal utilisé et de la valence des ligands L₁, L₂ et L₃ ainsi que de la charge des ligands L₁ et L₂.

2. Complexe métallo-organique selon la revendication 1, **caractérisé en ce que** le ligand L₁ présente une énergie de l'état triplet d'au moins 16 000 cm⁻¹, de préférence de 16 000 cm⁻¹ à 19 500 cm⁻¹.

3. Complexe métallo-organique selon la revendication 1 ou 2, **caractérisé en ce que** les radicaux et indices dans le dérivé de triphénylène de formule (II) présentent les significations suivantes R², R³, R⁴ indépendamment l'un de l'autre, C₁-C₂₀-alkyle, C₀-C₂₀-alkylène-C₃-C₁₈-cycloalkyle, C₀-C₂₀-alkylène-hétérocycloalkyle comprenant 3 à 18 atomes de cycle, C₀-C₂₀-alkylène-C₁-C₂₀-alcoxy, C₀-C₂₀-alkylène-C₆-C₁₈-aryloxy, C₀-C₂₀-alkylène-C₆-C₁₈-aryle, C₀-C₂₀-alkylène-hétéroaryle comprenant 5 à 18 atomes de cycle, où les radicaux susmentionnés peuvent être substitués par hydroxy, halogène, alkyle, cycloalkyle, hétérocycloalkyle, aryle, hétéroaryle ou amino, ou non substitués ; pseudohalogène ou halogène ;
O 0 à 3, où les radicaux R³, lorsque o > 1, peuvent être identiques ou différents ;
p 0 à 2, où les radicaux R⁴, lorsque p > 1, peuvent être identiques ou différents ; n 0 ;
X² CH ;
X³ CH ou CR³ ;
X⁴ indépendamment l'un de l'autre, CH ou CR⁴.

4. Complexe métallo-organique selon l'une quelconque des revendications 1 à 3, **caractérisé en ce que** l'atome métallique M est Ir (III).

5. Complexe métallo-organique selon l'une quelconque des revendications 1 à 4, **caractérisé en ce que**
M représente Ir (III) ;
L₂ représente un ligand bidentate monoanionique ;
q vaut 1 ou 2 ;
r vaut 1 ou 2 ;
s vaut 0,
et la somme q + r = 3.

6. Complexe métallo-organique selon l'une quelconque des revendications 1 à 5, **caractérisé en ce que** L₂ est choisi dans le groupe constitué par les β-dicétonates tels que l'acétylacétonate et ses dérivés, le picolinate, les ions d'acides aminés et les ligands bidentates monoanioniques de formule générale (b) 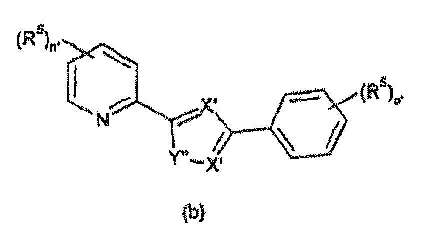 dans laquelle
R⁵ signifie hydrogène, C₁-C₆-alkyle, C₀-C₄-alkylène-C₃- C₈-cycloalkyle, C₀-C₄-alkylène-C₆-C₁₈-aryle ;
X' signifie N ;
Y" signifie N ;
n' vaut 1, 2, 3 ou 4 ; et
O' vaut 1, 2, 3, 4 ou 5.

7. Procédé pour la préparation de complexes métallo-organiques selon l'une quelconque des revendications 1 à 6 par
(a) transformation de sels ou de complexes d'iridium, qui contiennent le cas échéant un ou plusieurs ligands L₃, avec un premier ligand L₁ ou L₂ en complexes d'iridium, qui portent soit un ou plusieurs ligands L₁, soit un ou plusieurs ligands L₂, le cas échéant en plus d'un ou de plusieurs ligands L₃ ;
(b) transformation des complexes d'iridium obtenus dans l'étape (a) avec un deuxième ligand L₁, lorsque le complexe d'iridium obtenu dans l'étape (a) contient un ou plusieurs ligands L₂ ou, selon le cas, avec un deuxième ligand L₂, lorsque le complexe d'iridium obtenu dans l'étape (a) contient un ou plusieurs ligands L₁, avec obtention d'un complexe métallo-organique de formule (I), où l'étape (b), dans le cas où le complexe métallo-organique de formule (I) ne contient pas de ligands L₂, c'est-à-dire lorsque r dans le complexe métallo-organique de formule (I) vaut 0, est supprimée.

8. Mélange contenant au moins un matériau de matrice et au moins un complexe métallo-organique selon l'une quelconque des revendications 1 à 6 ou préparé selon la revendication 7.

9. Utilisation de complexes métallo-organiques selon l'une quelconque des revendications 1 à 6 ou préparés selon la revendication 7 ou de mélanges selon la revendication 8 dans des diodes organiques luminescentes.

10. Utilisation selon la revendication 8 ou 9, **caractérisée en ce que** les complexes métallo-organiques sont utilisés comme matériaux d'émission.
